(19) Europäisches Patentamt
European Patent Office
Office européen des brevets

(11) Veröffentlichungsnummer: **0 054 839**
**B1**

(12) # EUROPÄISCHE PATENTSCHRIFT

(45) Veröffentlichungstag der Patentschrift: **19.06.85**

(21) Anmeldenummer: **81110275.5**

(22) Anmeldetag: **09.12.81**

(51) Int. Cl.⁴: **C 07 D 243/16,** A 61 K 31/55, C 07 D 491/02 // (C07D491/02, 243:00, 317:00),(C07D491/02, 243:00, 319:00)

(54) **2-Acylaminomethyl-1,4-benzodiazepine und deren Salze sowie Verfahren zu ihrer Herstellung und diese Verbindungen enthaltende Arzneimittel.**

(30) Priorität: **20.12.80 DE 3048264**

(43) Veröffentlichungstag der Anmeldung: **30.06.82 Patentblatt 82/26**

(45) Bekanntmachung des Hinweises auf die Patenterteilung: **19.06.85 Patentblatt 85/25**

(84) Benannte Vertragsstaaten: **AT BE CH DE FR GB IT LI LU NL SE**

(56) Entgegenhaltungen:
**BE - A - 799 001**
**DE - A - 2 221 558**
**DE - A - 2 353 187**
**DE - A - 2 436 147**
**DE - A - 2 952 279**

***Die Akte enthält technische Angaben, die nach dem Eingang der Anmeldung eingereicht wurden und die nicht in dieser Patentschrift enthalten sind.***

(73) Patentinhaber: **Kali-Chemie Pharma GmbH, Hans-Böckler-Allee 20 Postfach 220, D-3000 Hannover 1 (DE)**

(72) Erfinder: **Zeugner, Horst, Dipl.-Chem. Dr. rer. nat., Havelweg 10, D-3000 Hannover 73 (DE)**
Erfinder: **Römer, Dietmar, Dr. rer. nat., Lettenweg 11, CH-4123 Allschwil (CH)**
Erfinder: **Benson, Werner, Dr. Dipl.-Chem., In der Steinbreite 39, D-3000 Hannover 91 (DE)**
Erfinder: **Liepmann, Hans, Dr. Dipl.-Chem., Auf dem Emmerberg 17, D-3000 Hannover 1 (DE)**
Erfinder: **Milkowski, Wolfgang, Dr. Dipl.-Chem., Fasanenweg 13, D-3167 Burgdorf (DE)**

(74) Vertreter: **Lauer, Dieter, Dr., c/o Kali-Chemie Aktiengesellschaft Postfach 220, D-3000 Hannover 1 (DE)**

**EP 0 054 839 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue 2-Phenylacylaminomethyl-5-phenyl-1,4-benzodiazepin-Verbindungen, deren Salze sowie Verfahren zu ihrer Herstellung und diese Verbindungen enthaltende pharmazeutische Zubereitungen.

Aus dem belgischen Patent Nr. 799 001 und den Teilen dieses belgischen Patentes entsprechenden deutschen Offenlegungsschriften 2 221 558 und 2 353 187 sind in 2-Stellung einen substituierten Methylrest tragende 5-Phenyl-1,4-benzodiazepine, u. a. 2-Acylaminomethyl-5-phenyl-1,4-benzodiazepine, in welchen der Acylrest ein niedermolekularer Alkanoylrest oder ein Trimethoxybenzoylrest ist, bekannt. Diese Substanzen besitzen vor allem zentraldepressive und antikonvulsive Wirkungen.

Der Erfindung liegt die Aufgabe zugrunde, neue 2-Acylaminomethyl-1,4-benzodiazepine mit neuartigem pharmakologischem Wirkungsprofil zu entwickeln.

Die erfindungsgemäße Gruppe von neuen 2-Phenylacylaminomethyl-5-phenyl-1,4-benzodiazepinen fällt unter den Umfang der in dem belgischen Patent 799 001 enthaltenen allgemeinen Formel.

Überraschenderweise wurde nun gefunden, daß die erfindungsgemäßen 2-Phenyl-1,4-benzodiazepine vor allem ausgeprägte analgetische Wirkungen neben sedierenden, diuretischen und antiarrhythmischen Wirkungen bei geringer Toxizität aufweisen.

Die vorliegende Verbindung betrifft daher 2-Phenylacylaminomethyl-1H-2,3-dihydro-1,4-benzodiazepine der Formel I

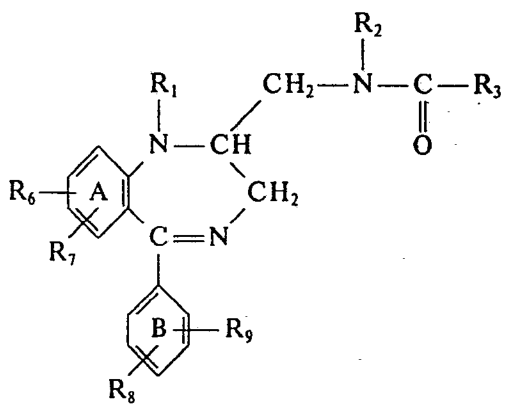

(I)

worin

$R_1$ Wasserstoff, Alkyl mit 1—4 Kohlenstoffatomen, Allyl, 2-Butenyl, 3-Butenyl oder Cyclopropylmethyl bedeutet,

$R_2$ Wasserstoff, Alkyl mit 1—4 Kohlenstoffatomen, Allyl, 2-Butenyl oder 3-Butenyl bedeutet,

$R_3$ eine Gruppe der Formel

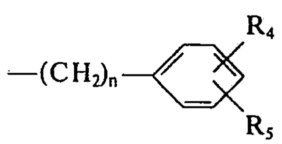

bedeutet, worin

$R_4$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituiertes Amino, Monoalkanoylamino mit 1—4 Kohlenstoffatomen oder Alkanoyloxy mit 1—4 Kohlenstoffatomen bedeutet, und

$R_5$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder

$R_4$ und $R_5$ an benachbarte Kohlenstoffatome gebunden sind und zusammen Methylendioxy oder Äthylendioxy bedeuten,

n 0, 1 oder 2 bedeutet,

$R_6$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituiertes Amino, Monoalkanoylamino mit 1—4 Kohlenstoffatomen oder Alkanoyloxy mit 1—4 Kohlenstoffatomen bedeutet, und

$R_7$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder

$R_6$ und $R_7$ an benachbarte Kohlenstoffatome gebunden sind und zusammen Methylendioxy oder Äthylendioxy bedeuteten,

$R_8$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituiertes Amino, Monoalkanoylamino mit 1—4 Kohlenstoffatomen oder Alkanoyloxy mit 1—4 Kohlenstoffatomen bedeutet, und

$R_9$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder

$R_8$ und $R_9$ an benachbarte Kohlenstoffatome gebunden sind und zusammen Methylendioxy oder Äthylendioxy bedeuten,

sowie deren optische Isomere und deren Säureadditionssalze.

Als Alkyl- und Alkenylgruppen $R_1$ und $R_2$ kommen niedermolekulare Gruppen mit 1—4 Kohlenstoffatomen in Frage, die gerade oder verzweigt sind, z. B. Methyl, Äthyl, Propyl, Isopropyl, n-Butyl, 1-Methylpropyl, 2-Methylpropyl, tert.-Butyl, Allyl, 2-Butenyl oder 3-Butenyl. Der Substituent $R_1$ steht insbesondere für Wasserstoff, niederes Alkyl oder Cyclopropylmethyl, vorzugsweise für niederes Alkyl, insbesondere Methyl. $R_2$ stellt bevorzugt Wasserstoff dar.

Sofern in den Verbindungen der Formel I die Substituenten $R_4$ und $R_5$, die Substituenten $R_6$ und $R_7$ des Phenylringes A oder die Substituenten $R_8$ und $R_9$ des Phenylringes B eine niedere Alkylgruppe darstellen oder enthalten, kann diese gerade oder verzweigt sein und 1—4 Kohlenstoffatome enthalten. So kommen insbesondere Methyl, Äthyl, n-Propyl, Isopropyl, n-Butyl, 1-Methylpropyl, 2-Methylpropyl oder tert.-Butyl, bevorzugt Methyl, Äthyl, n-Propyl und Isopropyl, in Frage. Insbesondere bei Disubstitionen an den Phenylringen sind Äthyl und insbesondere Methyl bevorzugt. Niedermolekulare Alkoxy- oder Alkylthiosubstituenten stellen vorzugsweise Methoxy oder Methylthio dar.

Für die Substituenten $R_4$ bis $R_9$ kommen als Halogenatome insbesondere Fluor, Chlor oder Brom infrage. Bei Substitution eines Phenylringes mit Alkylthio, Nitro, Trifluormethyl, Cyano, Amino und substiertem Amino ist die Monosubstitution bevorzugt.

Erfindungsgemäß werden die 2-Phenylacylaminomethyl-1H-2,3-dihydro-1,4-benzodiazepin-Verbindungen der Formel I sowie deren optische Isomere und Säureadditionssalze erhalten, indem man in an sich bekannter Weise 2-Aminomethyl-1H-2,3-dihydro-1,4-benzodiazepin-Verbindungen der Formel II

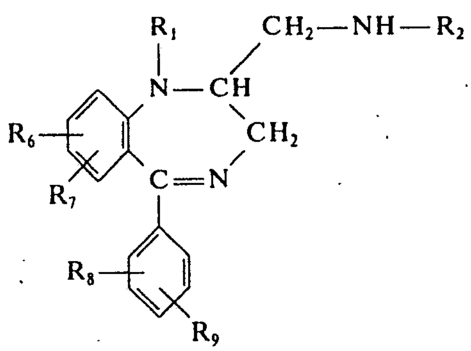

(II)

worin $R_1$, $R_2$, $R_6$, $R_7$, $R_8$ und $R_9$ die oben angegebenen Bedeutungen haben, oder deren Säureadditionssalze mit einer Carbonsäure oder einem reaktiven Carbonsäurederivat der Formel III,

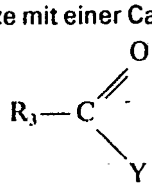

(III)

worin $R_3$ die obengenannte Bedeutung hat und Y Hydroxy, Halogen, niederes Alkoxy oder eine O—CO—Z-Gruppe bedeutet, worin Z die Bedeutung $R_3$ oder niederes Alkoxy hat, in einem inerten Lösungsmittel bei Temperaturen zwischen —30° C und dem Siedepunkt des eingesetzten Lösungsmittels acyliert, und gegebenenfalls Verbindungen der Formel I, worin $R_2$ Wasserstoff bedeutet, zu Verbindungen der Formel I, worin $R_2$ niederes Alkyl oder Alkenyl bedeutet, alkyliert und/oder in Verbindungen der Formel I, worin $R_6$ und/oder $R_7$ Wasserstoff bedeuten, in den Phenylring Chlor, Brom, Hydroxy oder Nitro einführt, und/oder in Verbindung der Formel I, worin $R_4$, $R_5$, $R_6$, $R_7$, $R_8$ und/oder $R_9$ niederes Alkanoyloxy oder niederes Alkanoylamino bedeuten, diese zu Hydroxy oder Amino hydrolysiert, und gegebenenfalls racemische Gemische von Verbindungen der Formel I in ihre optischen Isomeren auftrennt und gegebenefalls freie Verbindungen der Formel I in ihre Säureadditionssalze überführt oder die Säureadditionssalze in die freien Verbindungen der Formel I überführt.

Die Acylierung der Aminomethyl-Verbindungen der Formel II kann nach an sich zur Bildung von

Amid-Gruppierungen durch Aminoacylierung üblichen Methoden ausgeführt werden. Die Acylierung kann in einem unter den Reaktionsbedingungen inerten Lösungsmittel bei Temperaturen zwischen —30° C und dem Siedepunkt des Lösungsmittels bei Normaldruck oder bei erhöhtem Druck erfolgen. Als Lösungsmittel eignen sich halogenierte Kohlenwasserstoffe wie Methylenchlorid oder Chloroform, aromatische Kohlenwasserstoffe wie Benzol, Toluol, Xylol oder Chlorbenzol, cyclische Äther wie Tetrahydrofuran oder Dioxan, Ketone wie z. B. Aceton oder Methylisobutylketon, oder Dimethylformamid oder Gemische dieser Lösungsmittel.

Bei Verwendung eines Carbonsäurehalogenids oder Carbonsäureanhydrids der Formel III als Acylierungsmittel wird die Umsetzung zweckmäßigerweise in Gegenwart eines säurebindenden Reagenzes durchgeführt. Als säurebindende Reagenzien eignen sich anorganische Basen wie z. B. Kaliumcarbonat, Natriumcarbonat oder Kaliumhydroxid, oder organische Basen, insbesondere tertiäre Niederalkylamine wie Triäthylamin, Tripropylamin oder Tributylamin und Pyridine wie Pyridin, 4-Dimethylaminopyridin oder 4-Pyrrolidinopyridin. Im Überschuß eingesetzt können die tertiären Amine zusätzlich als inerte Lösungsmittel dienen.

Bei Verwendung einer Verbindung III, in welcher Y die Bedeutung Halogen hat, eignen sich insbesondere solche Verbindungen mit Y gleich Chlor.

Bei Verwendung einer Verbindung III, in welcher Y die Bedeutung einer niedermolekularen Alkoxygruppe hat, wird die Umsetzung zweckmäßigerweise in einem geschlossenen Gefäß durchgeführt, wobei auch im Überschuß eingesetzter Ester als Lösungsmittel dienen kann. Die Umsetzung kann durch Zugabe eines Metallalkoholats katalysiert werden, z. B. durch Zusatz von Aluminiumisopropylat oder -trialkylen.

Falls als Acylierungsmittel die Säure selbst oder auch ein Ester eingesetzt wird, so wird die Umsetzung der Aminoverbindung der Formel II mit der Säure der Formel III oder auch deren Ester zweckmäßigerweise in Gegenwart eines geeigneten Kopplungsreagenzes durchgeführt. Zur Amidbildung geeignete Kopplungsreagenzien sind z. B. aus der Peptidchemie allgemein bekannt. Als Beispiele für Kopplungsreagenzien, welche die Amidbildung dadurch fördern, daß sie mit der Säure in situ unter Bildung eines reaktionsfähigen Säurederivates reagieren, seien insbesondere genannt N-Niederalkyl-2-halogenpyridiniumsalze, insbesondere Halogenide oder Tosylate, vorzugsweise N-Methyl-2-chlorpyridiniumjodid, (siehe z. B. Mukaiyama in Angew. Chem. 91 789—812 (1979)) und Alkyl-, insbesondere Cycloalkylcarbodiimide, vorzugsweise Dicyclohexylcarbodiimid oder Carbonyldiimidazol. Die Umsetzung in Gegenwart eines Kopplungsreagenzes kann zweckmäßig bei Temperaturen von —30 bis +30° C unter Benutzung von inerten organischen Lösungsmitteln wie halogenierten Kohlenwasserstoffen und/oder aromatischen Kohlenwasserstoffen gegebenenfalls in Gegenwart eines säurebindenden Amins durchgeführt werden. Weitere zur erfindungsgemäßen Amidbildung geeignete Kopplungsreagenzien, welche auch in Peptidsynthesen Anwendung finden, sind z. B. bekannt aus Advanced Organic Chemistry by Jerry March McGraw-Hill Ltd., 2. Ausgabe, Seite 382 bis 388, und aus The Chemistry of Amides by Jacob Zabicky 1970 Interscience Publishers John Wiley and Sons, London Chapter 2: Synthesis of Amides.

Wenn $R_4$ oder $R_5$ in Verbindungen der allgemeinen Formel III Amino-, monosubstituierte Amino- oder Hydroxygruppe sind, können diese Reste vor der Umsetzung mit einer Schutzgruppe versehen werden, welche nach der Reaktion ggf. hydrolytisch abgespalten werden kann. Die freien Amino-, Monoalkylamino oder Hydroxygruppen lassen sich besonders günstig als leicht wieder spaltbare Sulfinylimino-, Acetylalkylamino- oder Acetoxygruppen schützen.

Erhaltene Verbindungen der Formel I, worin $R_2$, die Bedeutung Wasserstoff hat, können nachträglich in die entsprechenden N-Alkylverbindungen übergeführt werden. Man kann z. B. eine Verbindung der Formel I mit $R_2$ gleich Wasserstoff in Gegenwart eines geeigneten inerten Lösungsmittels mit einem Metallierungsmittel, wie Natriumhydrid, Lithiumbutyl, Lithiumphenyl, Natriumamid, Lithiumdiisopropylamid, Natriumalkoxid oder Thallium-I-alkoxid, umsetzen und anschließend die metallierte Verbindung bei einer Temperatur von —80° C bis zur Siedetemperatur des eingesetzten Lösungsmittels mit einem Alkylhalogenid, Alkylsulfat oder Alkylsulfonsäureester umsetzen.

Inerte Lösungsmittel sind in Abhängigkeit vom verwendeten Metallierungsmittel Diäthyläther, Tetrahydrofuran, Dioxan, Benzol, Toluol, Dimethylformamid, Dimethylsulfoxid, für die Metallalkoxide aber auch die entsprechenden Alkohole, also für Methoxide Methanol und für Äthoxide Äthanol.

Die erfindungsgemäßen Verbindungen der Formel I werden bei der Synthese aus Racematen der Verbindungen der Formel II in Form ihrer Racemate erhalten. Unter den Schutz dieser Erfindung fallen nun sowohl die racemischen Gemische wie auch die optisch aktiven Formen. Die optisch aktiven Verbindungen können aus den racemischen Gemischen der allgemeinen Formel I in an sich bekannter Weise durch Salzbildung mit geeigneten optisch aktiven Säuren, wie beispielsweise Weinsäure, O,O'-Dibenzoylweinsäure, Mandelsäure, Di-O-isopropyliden-2-oxo-L-gulonsäure, und anschließende fraktionierte Kristallisation der gewonnenen Salze in ihre optisch aktiven Antipoden aufgetrennt werden (S. H. Willen, A. Collet, J. Jaques, Tetrahedron 33 (1977) 2725—2736). Aus diesen Salzen kann man die freien Basen gewinnen, und diese gewünschtenfalls in pharmakologisch verträgliche Salze überführen. Durch Umkristallisation aus Lösungsmitteln, wie niederen Alkoholen und/oder Äther, können die racemischen Gemische und ihre optisch aktiven Isomeren sowie deren Säureadditionssalze gereinigt werden.

Die Auftrennung in die optisch aktiven Verbindungen kann aber auch in einer geeigneten Vorstufe bei der Herstellung der Ausgangsverbindungen der Formel II erfolgen.

Die als Ausgangssubstanzen verwendeten 2-Aminomethyl-Verbindungen der Formel II sind bekannt. Ihre Herstellung kann in an sich bekannter Weise erfolgen, z. B. nach den in dem belgischen Patent Nr. 799 001 beschriebenen Verfahren.

Bevorzugt wird man ein Acyldiamin der allgemeinen Formel IV

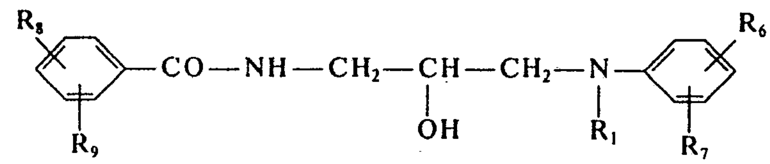

(IV)

in welcher $R_1$, $R_6$, $R_7$, $R_8$ und $R_9$ die obige Bedeutung haben, mit überschüssigem Phosphoroxychlorid mehrere Stunden unter Rückfluß erhitzen und anschließend das entstehende Isomerengemisch, bestehend aus den Verbindungen der allgemeinen Formel V und VI

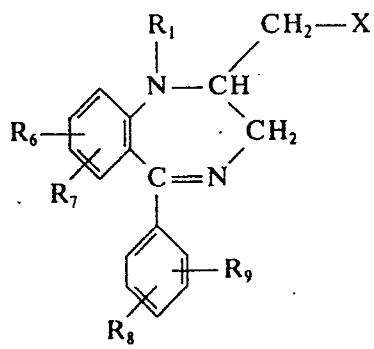

(V)

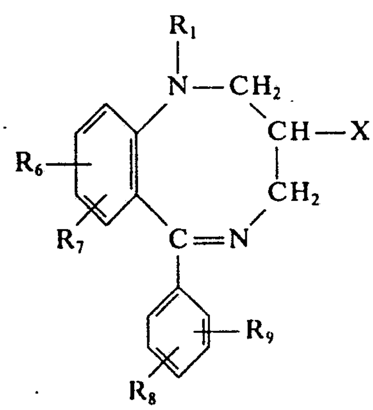

(VI)

worin $R_1$, $R_6$, $R_7$, $R_8$ und $R_9$ obige Bedeutung besitzen, aus dem Reaktionsgemisch abtrennen. Durch Umsetzung mit Ammoniak oder primären Aminen, ggf. in Gegenwart geeigneter Lösungsmittel, lassen sich daraus bei Temperaturen zwischen 20 und 150° C bei Normaldruck oder erhöhtem Druck direkt die 2-Aminomethyl-1,4-benzodiazepine der allgemeinen Formel II erhalten. In manchen Fällen kann es vorteilhaft sein, wenn man das Isomerengemisch der Verbindungen der allgemeinen Formel V und VI in an sich bekannter Weise mit einem Alkalimetallimid, vorzugsweise Kaliumphthalimid, in das 2-Phthalimidomethyl-1,4-benzodiazepinderivat bzw. mit einem Alkalimetallazid, wie Natrium- oder Kaliumazid, in das 2-Azidomethyl-1,4-benzodiazepinderivat überführt. In an sich bekannter Weise kann man beide Verbindungen zu den gewünschten 2-Aminomethyl-1,4-benzodiazepinen der allgemeinen Formel II spalten.

Eine nachträgliche Substitution im Phenylring des 1,4-Benzodiazepin-Systems ist sowohl auf der Stufe der cyclisierten Zwischenprodukte wie im Endprodukt in an sich bekannter Weise durch Halogen oder die Nitrogruppe möglich, beispielsweise nach den in dem belgischen Patent Nr. 799 001 beschriebenen Methoden. Als Halogenierungsmittel können beispielsweise N-Chlorsuccinimid oder N-Bromsuccinimid dienen. Zur Einführung der Nitrogruppe können die üblichen Nitrierungsreagenzien herangezogen werden, beispielsweise $KNO_3$ in $H_2SO_4$ oder als schonendes Nitrierungsreagenz Kupfer-II-Nitrat-Trihydrat in Acetanhydrid.

Sofern die Substituenten in dem Benzodiazepingerüst nicht Alkoxy- oder Alkylthiogruppen enthalten, können Ausgangsverbindungen der Formel II, worin $R_1$ Wasserstoff ist, auch dadurch erhalten werden, daß man Verbindungen der Formel II, worin $R_1$ Alkyl, vorzugsweise Methyl bedeutet, mit Jodwasserstoffsäure in an sich bekannter Weise entalkyliert. Die Reaktion kann in konzentrierter Jodwasserstoffsäure bei Temperaturen zwischen 50 und 100° C durchgeführt werden.

Die nach den erfindungsgemäßen Verfahren erhaltenen Verbindungen der allgemeinen Formel I werden entweder als freie Basen isoliert oder gewünschtenfalls in üblicher Weise in ihre Additionssalze mit anorganischen oder organischen Säuren überführt. Dazu versetzt man beispielsweise eine Lösung einer Vebindung der allgemeinen Formel I in einem geeigneten Lösungsmittel mit der als Salzkomponente gewünschten Säure. Vorzugsweise wählt man für die Umsetzung organische Lösungsmittel, in denen das entstehende Salz unlöslich ist, damit es durch Filtration abgetrennt werden kann. Solche Lösungsmittel sind beispielsweise Äthanol, Isopropanol, Äther, Aceton, Essigsäureäthylester, Aceton-Äther, Aceton-Äthanol, Äthanol-Äther.

Aus der Literatur ist bekannt, daß in 2-Stellung substituierte 1,4-Benzodiazepine wertvolle pharmakologische Eigenschaften aufweisen. Insbesondere beeinflussen die bekannten Benzodiazepin-Verbindungen das Zentralnervensystem (siehe belgisches Patent Nr. 799 001) und sind aufgrund ihrer anxiolytischen und aggressionsdämpfenden Eigenschaften als wertvolle Arzneimittel zur Behandlung dieser Symptomatik beim Menschen verwendbar. Es war nun umso überraschender, daß die neuen erfindungsgemäßen 2-Phenylacylaminomethyl-1,4-benzodiazepine ein pharmakologisch neuartiges Wirkungsprofil zeigen, wobei neben psychopharmakologischen, diuretischen und antiarrhythmischen Wirkungen ausgeprägte analgetische Eigenschaften vorherrschen. So zeigen die Verbindungen der Formel I in pharmakologischen Tests an Tieren in einem Dosierbereich von 0,1—100 mg/kg analgetische Wirkungen.

Aufgrund ihrer ausgeprägten analgetischen Eigenschaften sind die erfindungsgemäßen neuen Verbindungen nützliche Analgetica.

In pharmakologischen Tests an kleinen Nagern und an Affen kann nachgewiesen werden, daß die Verbindungen der Formel I die Schmerzschwelle bei Säugetieren heraufzusetzen vermögen. Dies wird insbesondere nachgewiesen in zwei pharmakologischen Standard-Testmethoden, dem Brennstrahltest an der Maus und dem Arthritisschmerztest an der Ratte.

## Beschreibung der pharmakologischen Untersuchungsmethoden

### 1. Bestimmung der minimalen toxischen Dosis

Bei männlichen Mäusen von 20—25 g Gewicht werden per os Maximaldosen von 300 mg/kg der Testsubstanz verabreicht. Die Tiere werden 3 Stunden lang sorgfältig auf Toxizitätssymptome beobachtet. Über einen Zeitraum von 24 Stunden nach der Applikation werden zusätzlich alle Symptome und Todesfälle registriert. Begleitsymptome werden ebenfalls beobachtet und registriert. Wenn Tod oder toxische Symptome beobachtet werden, werden weiteren Mäusen zunehmend geringere Dosen verabreicht, bis keine toxischen Symptome mehr auftreten. Die niedrigste Dosis, welche toxische Symptome hervorruft, wird als minimale toxische Dosis angegeben.

### 2. Arthritisschmerztest an der Ratte

Männliche Ratten vom Stamm OFA mit einem Gewicht von 160—180 g werden durch Gaben von 20 mg/kg i. p. Pentobarbitalnatrium anästhesiert. 0,1 ml einer Suspension von Mycobakterium Smegmae (SI 043) in Paraffinöl (0,6 mg Mycobakt./0,1 ml Öl) werden intracutan in die linke Hinterpfote injiziert. 14 Tage später, wenn sich besonders in der rechten Hinterpfote eine ausgeprägte sekundäre Arthritis entwickelt hat, werden die Wirkungen der Testsubstanzen untersucht. 30 Minuten vor Verabreichung der Testsubstanzen wird eine Kontrollmessung vorgenommen, indem das Fußgelenk der rechten Hinterpfote dreimal gebeugt wird und die Anzahl der Lautgebungen gezählt wird. Ratten, welche nicht reagieren, werden ausgesondert. 3 Stunden nach oraler Gabe der Testsubstanzen wird die Beugeprozedur wiederholt. Tiere, welche entweder nur einmal oder überhaupt nicht Laut geben, werden als geschützt gegen Schmerz angesehen. Zwischen 9 und 20 Ratten werden pro Dosis verwendet, und die $ED_{50}$ (95% Vertrauensbereich) wird nach der Methode von Litchfield und Wilcoxon (1949) bestimmt. Als $ED_{50}$ wird die Dosis bezeichnet, welche einen Schutz in 50% der behandelten Tiere erzeugt.

### 3. Brennstrahl-Test an der Maus (Tail flick test)

Die Methode basiert auf der von D'Amour und Smith beschriebenen Arbeitsweise (1941). Es werden jedoch gefütterte männliche und weibliche Mäuse mit 16—25 g Körpergewicht anstelle von Ratten verwendet. 30 Minuten vor der Behandlung mit der Testsubstanz wird jede Maus einzeln in ein zylindrisches Gefäß gesetzt, so daß sie sich nicht umdrehen und vorwärts bewegen kann. Ihr Schwanz ragt in einer engen Rinne liegend aus dem Gefäß heraus. Ein bestimmter Punkt des Schwanzes jedes Tieres (ungefähr 35 mm von der Schwanzwurzel entfernt) wird der Bestrahlungshitze einer Lampe bekannter Stärke und Temperatur ausgesetzt, die sich direkt unter dem Schwanz befindet. Die Zeit in Sekunden, die die Maus braucht, um den Schwanz aus dem Lichtstrahl zu schnellen, wird zweimal bestimmt, einmal 30 und einmal 15 Minuten vor subkutaner Verabreichung der Testsubstanz (10 mg/kg). Die Mäuse, deren Reaktionszeiten um mehr als 25% abweichen, werden ausgeschieden. Die Reaktionszeiten werden erneut nach 15 und 30 Minuten nach der Behandlung gemessen und eine Ausdehnung der Reaktionszeiten auf mehr als 75% der durchschnittlichen Vorbehandlungswerte derselben Maus wird als analgetischer Effekt angesehen. Als $ED_{50}$ (95% Vertrauensbereich) jeder Testsubstanz 30 Minuten nach Verabreichung wird die Dosis angesehen, welche die Vorbehandlungsreaktionszeit um mehr als 75% in 50% der Tiere verlängert. Berechnung erfolgt nach der Methode von Lichtfield und Wilcoxon (1949).

Die folgende Tabelle gibt nach den vorstehend beschriebenen Testmethoden erhaltene Ergebnisse wieder

Die für die Verbindungen der Formel I angegebenen Beispielsnummern beziehen sich auf die nachstehenden Herstellungsbeispiele.

| Test Substanz der Formel I Beispiel Nr. | Hemmung des Arthritis-Schmerzes an der Ratte ED50 mg/kg p. o. | Tail flick Test an der Maus ED50 mg/kg s. c. | minimale toxische Dosis an der Maus mg/kg p. o. |
|---|---|---|---|
| 18 | 7,5 | | >300 |
| 19 | ~2,3 | 1,0 | >300 |
| 20 | ~4 | 4,6 | 300 |
| 25 | 3,2 | 2,5 | |
| 28 | 0,6 | | >300 |
| 29 | 1,7 | 21 | 300 |
| 31 | <18>10 | 1,8 | >300 |
| 32 | 1,7 | 4,2 | 300 |
| 33 | <10>5,6 | 1,4 | >300 |
| 35 | 9 | 13 | |
| 40 | 11 | | >300 |
| 1 | 19 | 0,52 | >300 |
| 2 | 2,0 | 0,64 | 50 |
| 4 | 0,5 | 0,07 | 10 |
| 47 | ~13 | 5,6 | >300 |
| 49 | 2,5 | 0,56 | 100 |
| 55 | <18>10 | >5,6<10 | |
| 58 | ~18 | 1,6 | 300 |
| 59 | 9,7 | 1 | 300 |
| 60 | 3,5 | 0,32 | 300 |
| 112 | 10 | 20 | 50 |
| 113 | <32>18 | <5,6 | 100 |
| 117 | 6 | 13 | >300 |
| 154 | 2 | | |
| 155 | ~2 | | |

Zur Verwendung als Arzneistoffe können sowohl die freien Basen als auch deren pharmazeutische annehmbare Säureadditionsalze eingesetzt werden, d. h. Salze mit solchen Säuren, deren Anionen bei den in Frage kommenden Dosierungen nicht toxisch sind. Ferner ist es von Vorteil, wenn die als

Arzneistoffe zu verwendenden Salze gut kristallisierbar und nicht oder nur wenig hygroskopisch sind. Zur Salzbildung mit Verbindungen der allgemeinen Formel I eignen sich beispielsweise für diese Zwecke Chlorwasserstoffsäure, Bromwasserstoffsäure, Schwefelsäure, Phosphorsäure, Methansulfonsäure, Äthansulfonsäure, Benzolsulfonsäure, p-Toluolsulfonsäure, Citronensäure, Essigsäure, Milchsäure, Bernsteinsäure, Maleinsäure, Fumarsäure, Äpfelsäure, Weinsäure, Benzoesäure, Phenylessigsäure und Mandelsäure.

Die Verbindungen der Formel I können in pharmazeutischen Gebrauchsformen zur Behandlung von Schmerzen verabreicht werden, wobei die Dosierung der zu behandelnden Spezies und den individuellen Erfordernissen angepaßt wird. Im allgemeinen werden jedoch bei Testtieren schmerzhemmende Wirkungen mit Dosen zwischen 0,1 und 100 mg/kg erhalten. Zur Behandlung von Schmerzen bei Menschen und größeren Säugetieren sind z. B. Präparate mit 0,25 bis 50 mg Aktivsubstanz pro Einzeldosis geeignet. Parenterale Formulierungen werden im allgemeinen weniger Aktivsubstanz als Präparate zu oraler Verabreichung enthalten.

Die Verbindungen der Formel I können allein oder in Kombination mit pharmazeutisch anwendbaren Trägermaterialien und üblichem pharmazeutischen Hilfsstoffen in vielen Dosierungsformen eingesetzt werden. Zum Beispiel kann man feste Präparate wie Tabletten, Kapseln, Pulver, Granulate, Suppositorien, Dragees und dergleichen für die Verabreichung einsetzen. Feste Präparate können einen anorganischen Trägerstoff wie Talkum oder einen organischen Trägerstoff wie Milchzucker oder Stärke enthalten, sowie Zusätze von üblichen Hilfsstoffen, z. B. Gleitmittel wie Magnesiumstearrat. Flüssige Präparate wie Lösungen, Suspensionen oder Emulsionen können die üblichen Verdünnungsmittel wie Wasser, Paraffine, Suspensionsmittel wie Polyoxyäthylenglykol und dergleichen enthalten. Es können auch zusätzliche andere Bestandteile zugegeben werden wie Konservierungsmittel, Stabilisierungsmittel und Netzmittel.

Die nachfolgenden Beispiele erläutern die Herstellung der neuen Verbindungen der allgemeinen Formel I, sie sollen jedoch den Umfang der Erfindung in keiner Weise beschränken.

Die Strukturen der neuen Verbindungen werden durch spektroskopische Untersuchungen, insbesondere durch eine genaue Analyse der NMR-Spektren gesichert. Im IR-Spektrum wird die Amid—C=O-Bande im Bereich 1630—1650 $cm^{-1}$ bestimmt. In der Tabelle werden bei Salzformen eventuell eingeschlossene Mengen an Wasser, Aceton, Äthanol oder dgl. angeführt.

### Beispiel 1

1-Methyl-2-benzoylaminomethyl-5-phenyl-1H-2,3-dihydro-1,4-benzodiazepin

a) 50,5 g $N_1$-Benzoyl-$N_2$-methyl-$N_2$-phenyl-2-hydroxy-1,3-diaminopropan werden mit 250 ml Phosphoroxidchlorid 2,5 h unter Rückfluß gekocht. Nach üblicher Aufarbeitung des Reaktionsgemisches werden 48 g eines Gemisches aus 1-Methyl-2-chlormethyl-5-phenyl-1H-2,3-dihydro-1,4-benzodiazepin und 1-Methyl-3-chlor-6-phenyl-1,2,3,4-tetrahydrobenzodiazocin als Rückstand erhalten. Durch 22stündiges Kochen dieses Gemisches mit 34,6 g Kaliumphthalimid und 9,6 g Kaliumjodid in 350 ml Methanol werden 64,1 g 1-Methyl-2-phthalimidomethyl-5-phenyl-1H-2,3-dihydro-1,4-benzodiazepin erhalten. Dieses wird ohne weitere Reinigung mit 17,8 g Hydrazinhydrat in 800 ml Äthanol unter Rückfluß erhitzt. Das erhaltene 1-Methyl-2-aminomethyl-5-phenyl-1H-2,3-dihydro-1,4-benzodiazepin wird auf übliche Weise in sein Dihydrochlorid mit Fp. 209—213°C überführt.

b) 19,3 g des vorstehenden Dihydrochlorids werden in 260 ml Methylenchlorid unter Zugabe von 26 ml Triäthylamin gelöst. Unter Eiskühlung wird eine Lösung von 7,3 ml Benzoylchlorid in 40 ml Methylenchlrorid zugetropft. Die Reaktionslösung wird noch 2 h bei Raumtemperatur gerührt und anschließend mit 100 ml Wasser, 100 ml 20%iger Ammoniaklösung, 50 ml Wasser und zweimal mit 50 ml gesättigter Natriumchloridlösung gewaschen. Die organische Phase wird über Natriumsulfat getrocknet und filtriert. Das Filtrat wird im Vakuum vom Lösungsmittel befreit. Als Rückstand werden 16,3 g der rohen Titelbase erhalten. Diese wird in Äther gelöst und durch Zugabe einer Lösung von Chlorwasserstoff in Äther das Hydrochlorid ausgefällt. Die Kristalle werden abfiltriert und mehrmals mit heißem Aceton ausgerührt. Es werden 8,4 g 1-Methyl-2-benzoylaminomethyl-5-phenyl-1H-2,3-dihydro-1,4-benzodiazepin-dihydrochlorid in Form gelber Kristalle mit Fp. 217—218°C erhalten.

### Beispiel 2

8-Methoxy-1-methyl-2-benzoylaminomethyl-5-(4'-fluorphenyl)-1H-2,3-dihydro-1,4-benzodiazepin

10,0 g 8-Methoxy-1-methyl-2-aminomethyl-5-(4'-fluorphenyl)-1H-2,3-dihydro-1,4-benzodiazepin werden in 200 ml Methylenchlorid gelöst und mit 5,26 ml Triäthylamin versetzt. Dann wird bei —5°C eine Lösung von 3,8 ml Benzoylchlorid in 50 ml Methylenchlorid zugetropft. Nach Aufarbeitung des

Reaktionsproduktes werden 6,8 g des 8-Methoxy-1-methyl-2-benzoylaminomethyl-5-(4'-fluorphenyl)-1H-2,3-dihydro-1,4-benzodiazepin-dihydrochlorids · 0,8 Mol $H_2O$ mit Fp. 219—222°C erhalten.

## Beispiel 3

### 7-Brom-1-methyl-2-(3-aminobenzoylaminomethyl)-5-(2'-chlorphenyl)-1H-2,3-dihydro-1,4-benzodiazepin

14,4 g 7-Brom-1-methyl-2-aminomethyl-5-(2'-chlorphenyl)-1H-2,3-dihydro-1,4-benzodiazepin werden in 200 ml Methylenchlorid gelöst und mit 6,1 ml Triäthylamin versetzt. Dann wird eine Lösung von 8,5 g 3-Sulfinyliminobenzoesäurechlorid (erhalten durch Umsetzung von Thionylchlorid mit 3-Aminobenzoesäure) in 50 ml Methylenchlorid unter Kühlung mit Eis zugetropft. Anschließend wird die Reaktionslösung über Nacht bei Raumtemperatur gerührt. Zur Aufarbeitung wird die Methylenchloridphase mit verdünnter Salzsäure (12%) ausgeschüttelt, anschließend durch Zugabe von Natronlauge (50%) die Base ausgeschieden und mit Methylenchlorid extrahiert. Nach üblichem Aufarbeiten werden 17,1 g rohe Titelbase erhalten. Diese wird zur groben Reinigung in ihr Dihydrochlorid überführt. Nach Rückverwandlung des Salzes in die Base (8,5 g) wird diese dünnschichtchromatographisch über Kieselgel mit einem Laufmittel aus Chloroform, Äthanol und konzentrierte Ammoniaklösung (90/5/1 Volumenteile) nochmals gereinigt. Das aus der gereinigten Base mit einer Ausbeute von 4 g erhaltene Dihydrochlorid × 1 Mol $C_2H_5OH$ × 1 Mol $H_2O$ hat einen Fp. 231—235°C.

## Beispiel 4

### 8-Methoxy-1-methyl-2-benzoylaminomethyl-5-(2',4'-dichlorphenyl)-1H-2,3-dihydro-1,4-benzodiazepin

12,8 g Benzoesäure werden in 300 ml Methylenchlorid gelöst, auf 0 bis 5°C abgekühlt und mit 24,6 ml Triäthylamin versetzt. Dann werden innerhalb von 5 bis 10 Minuten 10 ml Chlorameisensäureäthylester zugetropft. Die Reaktionslösung wird noch 30 Minuten bei einer Temperatur zwischen 0 und 5°C gerührt und dann unter Kühlung und Feuchtigkeitsausschluß so zu einer Lösung von 38,2 g 8-Methoxy-1-methyl-2-aminomethyl-5-(2',4'-dichlorphenyl)-1H-2,3-dihydreo-1,4-benzodiazepin in 200 ml Methylenchlorid getropft, daß die Temperatur zwischen 0 und 5°C bleibt. Die Reaktionslösung wird noch 4 h bei Raumtemperatur gerührt. Nach Aufarbeitung des Reaktionsproduktes werden 50,3 g rohe Titelverbindung erhalten, aus welcher das Hydrochlorid mit einem Fp. 246—248°C in einer Ausbeute von 35,5 g erhalten wird.

## Beispiel 5

### 1-Methyl-2-(3-methoxybenzoylaminomethyl)-5-phenyl-1H-2,3-dihydro-1,4-benzodiazepin

9,19 g 2-Chlor-1-methylpyridiniumjodid werden in 300 ml Methylenchlorid bei Raumtemperatur unter Rühren und unter Feuchtigkeitsausschluß aufgeschlämmt und anschließend mit 10 ml Triäthylamin und 4,56 g 3-Methoxybenzoesäure versetzt. Nach 15 Minuten wird eine Lösung von 7,7 g 1-Methyl-2-aminomethyl-5-phenyl-1H-2,3-dihydro-1,4-benzodiazepin in Methylenchlorid innerhalb von 15 Minuten zugetropft. Nach weiteren 30 Minuten Rühren werden 300 ml Wasser und wenig wäßrige Ammoniaklösung bis zur schwachen ammoniakalischen Reaktion hinzugefügt. Aus der Methylenchloridphase werden 12,5 g öliger Rückstand erhalten, der nacheinander mit Äther, Methylenchlorid und Äthanol an 150 g Kieselgel (technisch) chromatographiert wird. Das aus der so erhaltenen Titelverbindung gewonnene Hydrochlorid hat nach Umkristallisieren aus Isopropanol einen Fp. 205—210°C. Die Ausbeute beträgt 10,8 g.

## Beispiel 6

### 1-Methyl-2-(2-hydroxybenzoylaminomethyl)-5-phenyl-1H-2,3-dihydro-1,4-benzodiazepin

Entsprechend dem Verfahren des Beispiels 5 wird aus 15 g 1-Methyl-2-aminomethyl-5-phenyl-1H-2,3-dihydro-1,4-benzodiazepin und 10 g Acetylsalicylsäure das 1-Methyl-2-[(2-acetoxybenzoyl)-aminomethyl]-5-phenyl-1H-2,3-dihydro-1,4-benzodiazepin dargestellt, das durch Behandeln mit 20%iger NaOH in Methanol in 30 Minuten zur Titelverbindung verseift wird. Das nach Extraktion mit Methylenchlorid als öliger Rückstand gewonnene Rohprodukt wird in Isopropanol aufgenommen und durch Einleiten von Chlorwasserstoff in das Hydrochlorid übergeführt, das durch Zugabe von Äther ausgefällt

wird. Nach Umkristallisieren aus Isopropanol/Methanol werden 12,2 g des Hydrochlorids mit Fp. 221—224°C erhalten.

## Beispiel 7

### 7-Brom-1-methyl-2-(2-fluorbenzoylaminomethyl)-5-(2'-fluorphenyl)1H-2,3-dihydro-1,4-benzodiazepin

30,3 g eines Gemisches aus 1-Methyl-2-chlormethyl-5-(2'-fluorphenyl)-1H-2,3-dihydro-1,4-benzodiazepin und 1-Methyl-3-chlor-6-(2'-fluorphenyl)-1,2,3,4-tetrahydro-benzodiazocin (erhalten durch Cyclisiuerung von $N_1$-(2-Fluorbenzoyl)-$N_2$-methyl-$N_2$-phenyl-2-hydroxy-1,3-diaminopropan mit $POCl_3$) werden in 300 ml Methylenchlorid mit 17,8 g N-Bromsuccinimid 24 h unter Rückfluß gekocht. Das bromierte Produkt wird in üblicher Weise mit Kaliumphthalimid in Methanol umgesetzt zu 1-Methyl-2-phthalimidomethyl-7-brom-5-(2'-fluorphenyl)-1H-2,3-dihydro-1,4-benzodiazepin. Nach Spaltung mit Hydrazinhydrat werden 17,3 g 7-Brom-1-methyl-2-aminomethyl-5-(2'-fluorphenyl)-1H-2,3-dihydro-1,4-benzodiazepin erhalten. Diese Verbindung wird mit 6,7 ml Triäthylamin in 250 ml Methylenchlorid gelöst und mit 7,6 g 2-Fluorbenzoylchlorid umgesetzt. Nach üblichem Aufarbeiten wird die erhaltene Titelbase in das Hydrochlorid mit Fp. 238—242° C überführt. ausbeute 12,3 g.

## Beispiel 8

### 2-Benzoylaminomethyl-5-phenyl-1H-2,3-dihydro-1,4-benzodiazepin

10 g 1-Methyl-2-aminomethyl-5-phenyl-1H-2,3-dihydro-1,4-benzodiazepin werden in 40 ml 67%iger Jodwasserstoffsäure unter Rühren 4 h auf 80°C erwärmt. Anschließend wird die Reaktionslösung durch Zusatz von 500 g Eis und festem Natriumcarbonat neutralisiert. Nach Zugabe von 50 ml konzentrierter Natronlauge wird die Mischung mit Methylenchlorid extrahiert. Nach üblichem Aufarbeiten der Methylenchloridphase werden 9 g 2-Aminomethyl-5-phenyl-1H-2,3-dihydro-1,4-benzodiazepin in Form eines Öles erhalten. Diese werden mit 4,2 ml Triäthylamin in 150 ml Methylenchlorid gelöst und mit 4,2 g Benzoylchlorid umgesetzt. Es werden 7,1 g 2-Benzoylaminomethyl-5-phenyl-1H-2,3-dihydro-1,4-benzodiazepin als öliges Rohprodukt erhalten. Dieses wird aus Methylenchlorid/Äther kristallisiert als Base · 0,005 HCl, Fp. 168—169°C.

## Beispiel 9

### 7-Nitro-1-methyl-2-benzoylaminomethyl-5-phenyl-1H-2,3-dihydro-1,4-benzodiazepin

Eine Lösung von 11,1 g 1-Methyl-2-chlormethyl-5-phenyl-1H-2,3-dihydro-1,4-benzodiazepin in 100 ml Eisessig wird mit 30 ml konzentrierter Schwefelsäure und anschließend bei 5°C mit einer Lösung von 9,6 g Kaliumnitrat in 21 ml konzentrierter Schwefelsäure versetzt. Das Reaktionsgemisch wird eine Stunde nachgerührt, auf Eis gegossen, mit verdünnter Natronlauge versetzt und mit Chloroform extrahiert. Nach üblicher Aufarbeitung werden 4,3 g 7-Nitro-2-chlormethyl-5-phenyl-1H-2,3-dihydro-1,4-benzodiazepin erhalten (Fp. des Hydrochlorids 212—215°C), die durch Umsetzung mit Kaliumphthalimid und Spaltung des Reaktionsproduktes mit Salzsäure (24%) in 7-Nitro-1-methyl-2-aminomethyl-5-phenyl-1H-2,3-dihydro-1,4-benzodiazepin übergeführt werden.

Dieses wird mit Triäthylamin und Benzoylchlorid in Methylenchlorid umgesetzt. Nach Aufarbeitung des Reaktionsgemisches erhält man 1,1 g 7-Nitro-1-methyl-2-benzoylaminomethyl-5-phenyl-1H-2,3-dihydro-1,4-benzodiazepin als Öl. Dieses wird in sein Hydrochlorid mit Fp. 212—215°C überführt.

## Beispiel 10

### 1-Methyl-2-[benzoyl-(N-methyl)-aminomethyl]-5-phenyl-1H-2,3-dihydro-1,4-benzodiazepin

9,3 g 1-Methyl-2-benzoylaminomethyl-5-phenyl-1H-2,3-dihydro-1,4-benzodiazepin werden in 100 ml Tetrahydrofuran gelöst und unter Rühren bei Raumtemperatur mit 0,75 g Natriumhydrid (80% in Öl) versetzt. Anschließend wird bei 5°C in die Reaktionsmischung langsam eine Lösung von 1,55 ml Methyljodid in 10 ml Tetrahydrofuran eingetropft. Die Reaktionsmischung wird 2 h bei 5—10°C gerührt und nach Zugabe von Toluol und Eiswasser wie üblich aufgearbeitet, wobei 7,2 g 1-Methyl-2-[benzoyl-(N-methyl-)-aminomethyl]-5-phenyl-1H-2,3-dihydro-1,4-benzodiazepin in Form einer öligen Base erhalten werden.

Beispiel 11

1-Methyl-2-(2-chlorphenacetylaminomethyl)-5-phenyl-1H-2,3-dihydro-1,4-benzodiazepin

Eine Mischung von 33,8 g 1-Methyl-2-aminomethyl-5-phenyl-1H-2,3-dihydro-1,4-benzodiazepin-dihydrochlorid und 25,1 g Triäthylamin in 460 ml Methylenchlorid wird unter Kühlung mit Eis tropfenweise mit einer Lösung von 23,3 g 2-Chlorphenacetylchlorid in 140 ml Methylenchlorid versetzt. Die Reaktionsmischung wird nach dem Zutropfen noch 1 Stunde bei Raumtemperatur gerührt. Nach dem üblichen Aufarbeiten mit Eis/Wasser werden 54,2 g rohe Titelverbindung erhalten, welche mit 200 ml Äther und 50 g $\gamma$-Tonerde gereinigt wird: Aus der gereinigten Titelbase werden 17,5 g 1-Methyl-2-(2-chlorphenacetylaminomethyl)-5-phenyl-1H-2,3-dihydro-1,4-benzodiazepinhydrochlorid vom Fp. 163—164,5°C.

Beispiel 12

1-Methyl-2-(3-phenylpropionylaminomethyl)-5-phenyl-1H-2,3-dihydro-1,4-benzodiazepin

14,5 g 3-Phenylpropionsäure werden in 250 ml Methylenchlorid gelöst und mit 14,5 ml Triäthylamin versetzt. Dann werden unter Eiskühlung (Innentemperatur 2—5°C) 10 ml Chlorameisensäureäthylester zugetropft. Die Reaktionslösung wird noch 30 Minuten bei dieser Temperatur gerührt und dann mit einer Lösung von 26,5 g 1-Methyl-2-aminomethyl-5-phenyl-1H-2,3-dihydro-1,4-benzodiazepin in 200 ml Methylenchlorid so versetzt, daß die Temperatur bei 0 bis 5°C bleibt. Nach 4 h Rühren bei Raumtemperatur wird wie gewöhnlich aufgearbeitet und 21,5 g 1-Methyl-2-(3-phenylpropionylaminomethyl)-5-phenyl-1H-2,3-dihydro-1,4-benzodiazepin als ölige Base erhalten. Die Base wird in ihr Hydrochloridsalz überführt. Diese kristallisiert mit der Zusammensetzung 1 Mol Base · 1,8 Mol HCl · 0,5 Mol $H_2O$. Fp. 112—114°C.

Beispiel 13

1-Methyl-2-benzoylaminomethyl-5-phenyl-1H-2,3-dihydro-1,4-benzodiazepin

26,5 g 1-Methyl-2-aminomethyl-5-phenyl-1H-2,3-dihydro-1,4-benzodiazepin werden mit einem Überschuß an Benzoesäuremethylester (100 ml) 2 h auf 120°C erhitzt. Anschießend wird der überschüssige Ester und das bei der Reaktion gebildete Methanol im Vakuum langsam abdestiliert. Man erhält einen Rückstand von 36 g Rohprodukt. Nach üblichem Aufarbeiten und Überführung in das Hydrochlorid werden 24,7 g 1-Methyl-2-benzoylaminomethyl-5-phenyl-1H-2,3-dihydro-1,4-benzodiazepinhydrochlorid mit Fp. 217—218°C erhalten.

Beispiel 14

1-Methyl-2-[benzoyl-(N-n-propyl)-aminomethyl]-5-phenyl-1H-2,3-dihydro-1,4-benzodiazepin

15,4 g 1-Methyl-2-N-n-propylaminomethyl-5-phenyl-1H-2,3-dihydro-1,4-benzodiazepin werden in 250 ml Äther gelöst und mit 2,8 g Chinuclidin versetzt. Dann werden unter Eiskühlung 7,1 g Benzoylchlorid, gelöst in 100 ml Äther, zugetropft und die Reaktionsmischung nach Beendigung des Zutropfens sofort in üblicher Weise aufgearbeitet. Man erhält 11,2 g 1-Methyl-2-[benzoyl-(N-n-propyl)-aminomethyl]-5-phenyl-1H-2,3-dihydro-1,4-benzodiazepin als ölige Base.

Entsprechend den Beispielen 1 bis 14 können folgende Verbindungen der Formel I, worin $R_1$, $R_2$, $R_4$, $R_5$, n, $R_6$, $R_7$, $R_8$ und $R_9$ die in der nachstehenden Tabelle angegebene Bedeutung besitzen, durch Acylierung entsprechend substituierter 2-Aminomethyl-5-phenyl-1H-2,3-dihydro-1,4-benzodiazepinen mit entsprechenden substituierten Verbindungen der Formel III erhalten werden:

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 15 | $CH_3$ | H | H | H | H | H | 0 | 2-Cl | H | HCl | 195—197 |
| 16 | $CH_3$ | H | H | H | H | H | 0 | 2-F | H | HCl | 189—193 |
| 17 | $CH_3$ | H | H | H | H | H | 0 | 2-$OCH_3$ | H | HCl | 196—199 |
| 18 | $CH_3$ | H | H | H | H | H | 0 | 2,4-di-Cl | | HCl | 254—260 |
| 19 | $CH_3$ | H | H | H | H | H | 0 | 3,4-di-Cl | | HCl | 172—175 |
| 20 | $CH_3$ | H | H | H | H | H | 0 | 4-$OCH_3$ | H | HCl | 215—220 |
| 21 | $CH_3$ | H | H | H | H | H | 0 | 4-$CF_3$ | H | p-tos. | 169—174 |
| 22 | $CH_3$ | H | H | H | H | H | 0 | 4-OH | H | HCl | 260—265 |
| 23 | $CH_3$ | H | H | H | H | H | 0 | 3,4-di-$OCH_3$ | | HCl | 214—218 |
| 24 | $CH_3$ | H | H | H | H | H | 0 | 3,5-di-$OCH_3$ | | HCl | 215—217 |
| 25 | $CH_3$ | H | H | H | H | H | 0 | 4-$CH_3$ | H | HCl | 195—199 |
| 26 | $CH_3$ | H | H | H | H | H | 0 | 3-$CH_3$ | H | HCl | 199—201 |
| 27 | $CH_3$ | H | H | H | H | H | 0 | 2-$CH_3$ | H | HCl | 189—193 |
| 28 | $CH_3$ | H | H | H | H | H | 0 | 4-$NO_2$ | H | HCl | 216—220 |
| 29 | $CH_3$ | H | H | H | 2-F | H | 0 | 4-CN | H | HCl | 239—242 |
| 30 | $CH_3$ | H | H | H | 2-F | H | 0 | 2-$OCH_3$ | H | HCl · 0,2 $H_2O$ · 0,1 i-$C_3H_7OH$ | 213—216 |
| 31 | $CH_3$ | H | H | H | 2-F | H | 0 | 3-$OCH_3$ | H | HCl | 225—228 |
| 32 | $CH_3$ | H | H | H | 2-F | H | 0 | 4-$OCH_3$ | H | HCl · 0,4 i-$C_3H_7OH$ | 201—205 |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 33 | $CH_3$ | H | H | H | 2-F | H | 0 | 4-$CF_3$ | H | HCl | 184—188 |
| 34 | $CH_3$ | H | H | H | 2-F | H | 0 | 4-OH | H | HCl | 253—257 |
| 35 | $CH_3$ | H | H | H | 2-F | H | 0 | 3-CN | H | HCl | 185—188 |
| 36 | $CH_3$ | H | 8-$CH_3$ | H | 4-F | H | 0 | 4-$OCH_3$ | H | HCl | 255—259 |
| 37 | $CH_3$ | H | H | H | 2-F | H | 0 | 3-OH | H | Base | 200—201 |
| 38 | $CH_3$ | H | H | H | 2-F | H | 0 | 3,4-O—$CH_2$—O | | HCl | 190—192 |
| 39 | $CH_3$ | H | H | H | 4-F | H | 0 | 2-$OCH_3$ | H | HCl | 175—181 |
| 40 | $CH_3$ | H | H | H | 4-F | H | 0 | 4-$OCH_3$ | H | HCl | 147—154 |
| 41 | $CH_3$ | H | H | H | 4-F | H | 0 | 2-$CH_3$ | H | HCl | 125—128 |
| 42 | $CH_3$ | H | H | H | 4-F | H | 0 | 3-$CH_3$ | H | HCl | 199—203 |
| 43 | $CH_3$ | H | H | H | 4-F | H | 0 | 4-$CH_3$ | H | Base | 77—83 |
| 44 | $CH_3$ | H | H | H | H | H | 0 | 4-$OC_2H_5$ | H | 1,05 HCl | 196—201 |
| 45 | $CH_3$ | H | H | H | 2-F | H | 0 | 2-OH | H | HCl | 219—223 |
| 46 | $CH_3$ | H | 7-$CH_3$ | H | 2-F | H | 0 | 3,4-di-Cl | | HCl | 220—225 |
| 47 | $CH_3$ | H | 7-$CH_3$ | H | 2-F | H | 0 | H | H | HCl | 249—254 |
| 48 | $CH_3$ | H | 8-$OCH_3$ | H | 4-F | H | 0 | 2-Cl | H | HCl | 236—239 |
| 49 | $CH_3$ | H | 7,8-O—$CH_2$—O | | H | H | 0 | H | H | HCl | 253—262 |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 50 | $CH_3$ | H | 7,8-O—$CH_2$—O | | H | H | 0 | 2-Cl | H | HCl | 246—253 |
| 51 | $CH_3$ | H | 7,8-O—$C_2H_4$—O | | H | H | 0 | 3,4-di-Cl | | HCl | 263—266 |
| 52 | $CH_3$ | H | 7,8-O—$C_2H_4$—O | | H | H | 0 | H | H | HCl | 267—274 |
| 53 | $CH_3$ | H | 7-$OCH_3$ | | H | H | 0 | H | H | HCl | 252—257 |
| 54 | $CH_3$ | H | 8-$OCH_3$ | H | 4-$CF_3$ | H | 0 | H | H | HCl | 239—241 |
| 55 | $CH_3$ | H | 8-$OCH_3$ | H | 3-$CF_3$ | H | 0 | H | H | HCl | 211—213 |
| 56 | $CH_3$ | H | 8-$OCH_3$ | H | 2-F | H | 0 | 3,4-di-Cl | | HCl | 202—203 |
| 57 | $CH_3$ | H | 8-$OCH_3$ | H | 3-F | H | 0 | 3,4-di-Cl | | HCl | 215—217 |
| 58 | $CH_3$ | H | 8-$OCH_3$ | H | 3-F | H | 0 | H | H | HCl | 200—232 (Z) |
| 59 | $CH_3$ | H | 8-F | H | H | H | 0 | H | H | HCl | 214—217 |
| 60 | $CH_3$ | H | 8-$OCH_3$ | H | 2-F | H | 0 | H | H | HCl | 216—219 |
| 61 | $CH_3$ | H | H | H | 2-F | H | 1 | 2-F | H | Base | 146—147 |
| 62 | $CH_3$ | H | 7-Br | H | 2-Cl | H | 0 | 3-F | H | HCl | 243—245 |
| 63 | $CH_3$ | H | 7-Br | H | 2-Cl | H | 0 | 2-F | H | HCl | 215—217,5 |
| 64 | $CH_3$ | H | 7-Br | H | 2-Cl | H | 0 | 3,4-O—$CH_2$—O | | HCl | 250—251 |
| 65 | $CH_3$ | H | 7-Br | H | 2-Cl | H | 0 | 3,4-di-$OCH_3$ | | HCl | 237—239 |
| 66 | $CH_3$ | H | 7,8-di-$OCH_3$ | | 3,4-di-$OCH_3$ | | 0 | 2-F | H | HCl · $H_2O$ | 167—170 |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 67 | $CH_3$ | H | 7-Br | H | 2-Cl | H | 0 | 4-F | H | HCl | 226—229 |
| 68 | $CH_3$ | H | 7-Br | H | 2-Cl | H | 0 | 2-Cl | H | HCl | 205—208 |
| 69 | $CH_3$ | H | H | H | 2-F | H | 0 | 2,4-di-Cl | | HCl | 180—185 |
| 70 | $CH_3$ | H | H | H | 4-F | H | 0 | 4-F | H | HCl | 182—187 |
| 71 | $CH_3$ | H | 8-F | H | 2-F | H | 0 | H | H | HCl | 191—194 |
| 72 | $CH_3$ | H | 7-F | 8-$CH_3$ | H | H | 0 | H | H | HCl | 112—116 |
| 73 | $CH_3$ | H | 7-$CH_3$ | 8-F | H | H | 0 | H | H | HCl | 233—237 |
| 74 | $CH_3$ | H | H | H | 4-Br | H | 0 | 4-$C(CH_3)_3$ | H | Base | 174—175 |
| 75 | $CH_3$ | H | H | H | 4-Br | H | 0 | H | H | Base | 168—171 |
| 76 | $CH_3$ | H | H | H | 4-F | H | 0 | 3-$OCH_3$ | H | HCl | 199—204 |
| 77 | $CH_3$ | H | H | H | 4-F | H | 0 | 4-OH | H | 1,25 HCL · 0,65 $C_2H_5OH$ | 184—189 |
| 78 | $CH_3$ | H | H | H | 4-Br | H | 0 | 3,5-di-$CH_3$ | | | |
| 79 | $CH_3$ | H | 8-$CH_3$ | H | 2-F | H | 0 | H | H | Base | Ö |
| 80 | $CH_3$ | H | 8-$CH_3$ | H | 4-F | H | 0 | H | H | Base · 1 $H_2O$ | 91— 93 |
| 81 | $CH_3$ | H | 8-$CH_3$ | H | 4-F | H | 0 | 4-$CH_3$ | H | Base · 1 $H_2O$ | 93— 96 |
| 82 | $CH_3$ | H | 8-$CH_3$ | H | 2-$CH_3$ | H | 0 | H | H | HCl | 231—235 |
| 83 | $CH_3$ | H | H | H | 2-F | H | 0 | 3-F | 6-$NH_2$ | Base | Ö |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 84 | $CH_3$ | H | H | H | 2-F | H | 0 | 4-$NH_2$ | H | HCl | 115—119 |
| 85 | $CH_3$ | H | H | H | 4-F | H | 0 | 4-$NHCOCH_3$ | H | Base | 122—126 |
| 86 | $CH_3$ | H | H | H | 4-F | H | 0 | 4-$NHCH_3$ | H | HCl | 165—170 |
| 87 | $CH_3$ | H | H | H | 4-F | H | 0 | 4-$N(CH_3)_2$ | H | HCl | 162—166 |
| 88 | $CH_3$ | H | 8-$CH_3$ | H | 2,4-di-Cl | | 0 | H | H | HCl | 252—256 |
| 89 | $CH_3$ | H | H | H | 4-$CH_3$ | H | 0 | 4-F | H | HCl | 206—210 |
| 90 | $CH_3$ | H | 8-$OCH_3$ | H | 2-F | H | 0 | 4-$CH_3$ | H | HCl | 160—162 |
| 91 | $CH_3$ | H | 8-$OCH_3$ | H | 2-F | H | 0 | H | H | p-tos | 182—183 |
| 92 | $CH_3$ | H | 8-$OCH_3$ | H | 4-Br | H | 0 | H | H | HCl | 239—241 |
| 93 | $CH_3$ | H | H | H | 2,4-di-$CH_3$ | | 0 | H | H | HCl | 195—197 |
| 94 | $CH_3$ | H | H | H | 4-OH | H | 0 | 4-OH | H | HCl | 105—109 |
| 95 | n-$C_4H_9$ | H | 8-Br | H | 4-$OCH_3$ | H | 0 | 4-$NO_2$ | H | Base | Ö |
| 96 | n-$C_4H_9$ | H | 8-Br | H | 4-F | H | 0 | 4-$NO_2$ | H | HCl | 197—200 |
| 97 | $CH_3$ | H | 7-$CH_3$ | H | 4-F | H | 0 | H | H | HCl | 194—197 |
| 98 | $CH_3$ | H | 7-$CH_3$ | H | 4-F | H | 0 | 4-$OCH_3$ | H | HCl | 208—211 |
| 99 | $CH_3$ | H | 7-$CH_3$ | H | 2-F | H | 1 | H | H | Base | Ö |
| 100 | $CH_3$ | H | 7-$CH_3$ | H | 2-$CH_3$ | H | 0 | H | H | Base | Ö |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 101 | $CH_3$ | H | 7-$CH_3$ | H | H | H | 0 | H | H | Base | Ö |
| 102 | $CH_3$ | H | 8-$CH_3$ | H | H | H | 0 | H | H | HCl | 248—252 |
| 103 | $CH_3$ | H | H | H | H | H | 0 | 3-OH | 4-$CH_3$ | HCl | 173—178 |
| 104 | $CH_3$ | H | 8-$NO_2$ | H | H | H | 0 | 4-$CH_3$ | H | HCl | 241—245 |
| 105 | $CH_3$ | H | H | H | 2-F | 5-N-$O_2$ | 0 | H | H | 1,2 HCl · 0,2 $H_2O$ | 119—123 |
| 106 | $CH_3$ | H | H | H | H | H | 0 | 2-$OCOCH_3$ | H | Base | Ö |
| 107 | $CH_3$ | H | 8-OH | H | H | H | 0 | 4-$CH_3$ | H | 1.15 HCl : 0,25 $C_2H_5OH$ | 127—131 |
| 108 | $CH_3$ | H | H | H | 2-Cl | 6-F | 0 | 4-CN | H | HCl | 236—250 (Z) |
| 109 | $CH_3$ | H | H | H | 2-$CH_3$ | H | 0 | 4-CN | H | HCl · 0,35$(CH_3)_2CO$ · 0,15 $H_2O$ | 174—180 |
| 110 | $CH_3$ | H | H | H | H | H | 0 | 4-CN | H | HCl | 208—212 |
| 111 | $CH_3$ | H | 7-Cl | H | H | H | 1 | H | H | HCl | 218—220 |
| 112 | $CH_3$ | H | 7-Cl | H | H | H | 1 | 3,4-di-$OCH_3$ | | HCl | 192—195 |
| 113 | $CH_3$ | H | 7,8-O—$CH_2$—O | | H | H | 1 | 3,4-di-$OCH_3$ | | HCl | 178—180 |
| 114 | $CH_3$ | H | H | H | H | H | 1 | 4-Br | H | 1,15 HCl · 0,3 $H_2O$ | 194—197 |
| 115 | $CH_3$ | H | H | H | H | H | 1 | 4-Cl | H | HCl · 0,7 $H_2O$ | 98—102 |
| 116 | $CH_3$ | H | H | H | 4-Br | H | 1 | 4-Br | H | HCL · 0,3 $H_2O$ | 179—182 |
| 117 | $CH_3$ | H | H | H | 2-F | H | 1 | 2-Cl | H | HCl | 171—174 |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 118 | $CH_3$ | H | H | H | 4-F | H | 1 | 2-Cl | H | Base · 0,15 $H_2O$ | 143—145 |
| 119 | $CH_3$ | H | H | H | 2-F | H | 1 | 4-Cl | H | HCl · 0,6 $H_2O$ | 99—105 |
| 120 | $CH_3$ | H | H | H | 4-F | H | 1 | 3-Cl | H | Base | 141—145 |
| 121 | $CH_3$ | H | H | H | 4-F | H | 1 | 4-Cl | H | HCl | 205—209 |
| 122 | $CH_3$ | H | H | H | 2-F | H | 1 | H | H | 0,875 HCl · 1 $H_2O$ | 107—112 |
| 123 | $CH_3$ | H | H | H | 2-F | H | 1 | 3-Cl | H | Base | 169—170 |
| 124 | $CH_3$ | H | 7,8-di-$OCH_3$ | | H | H | 1 | 2-Cl | H | HCl | 211,5—212 |
| 125 | $CH_3$ | H | 7-Br | H | 2-Cl | H | 1 | 3,4-O-$C_2H_4$-O | | HCl | 177—179 |
| 126 | $CH_3$ | H | 7-Br | H | 2-Cl | H | 1 | 3,4-di-$OCH_3$ | | HCl | 190—191 |
| 127 | $CH_3$ | H | H | H | H | H | 1 | 3-Cl | H | 1,1 HCl · 0,6 $H_2O$ | 92— 96 |
| 128 | $CH_3$ | H | H | H | 2-F | H | 1 | 2,4-di-Cl | | Base · 0,15 HCl | 109—112 |
| 129 | H | H | H | H | H | H | 1 | H | | Base | Ö |
| 130 | $CH_3$ | H | 8-F | H | 2-F | H | 1 | H | H | HCl | 88— 90 |
| 131 | $CH_3$ | H | 7-F | 8-$CH_3$ | H | H | 1 | H | H | HCl · 0,4 $H_2O$ | 95— 99 |
| 132 | $CH_3$ | H | 7-$CH_3$ | 8-F | H | H | 1 | H | H | HCl | 120—125 |
| 133 | $CH_3$ | H | 8-$CH_3$ | H | 2-F | H | 1 | H | H | Base | 151—154 |
| 134 | $CH_3$ | H | 8-$CH_3$ | H | 4-F | H | 1 | 4-$OCH_3$ | H | 1,1 HCl · 0,4 $H_2O$ | 110—115 |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 135 | $CH_3$ | H | 8-$CH_3$ | H | 2,4-di-Cl | | 1 | H | H | HCl · 0,5 $H_2$ | 123—127 |
| 136 | $CH_3$ | H | 8-$OCH_3$ | H | 2-F | H | 1 | H | H | HCl · 0,5 $H_2O$ | 113—117 |
| 137 | $CH_3$ | H | 8-$OCH_3$ | H | 2-F | H | 1 | 2-Cl | H | 1,55 HCl · 0,3 $H_2O$ · 0,25 $(CH_3)_2CO$ | 123—128 |
| 138 | $C_2H_5$ | H | H | H | 2-F | H | 1 | 2-Cl | H | Base | 147—150 |
| 139 | $C_2H_5$ | H | H | H | 2-F | H | 1 | 2-F | H | Base | 90— 96 |
| 140 | n-$C_4H_9$ | H | H | H | 2-F | H | 1 | 2-Cl | H | Base | 174—176 |
| 141 | n-$C_3H_7$ | H | H | H | 2-F | H | 1 | 2-Cl | H | Base | 167—172 |
| 142 | $CH_3$ | H | H | H | H | H | 1 | 4-$OCH_3$ | H | Base | Ö |
| 143 | $CH_3$ | H | H | H | 2-$CH_3$ | H | 1 | 2-Cl | H | HCl · 0,1$(CH_3)_2CO$ · 0,05 $CH_3COOC_2H_5$ · 0,25 $H_2O$ | 110—115 |
| 144 | $CH_3$ | H | H | H | 4-F | H | 2 | 4-OH | H | 1,75 HCl · 0,4 $H_2O$ | 125—128 |
| 145 | $CH_3$ | H | H | H | H | H | 2 | 4-OH | H | 1,9 HCl · 0,9 $H_2O$ | 136—139 |
| 146 | $CH_3$ | H | H | H | 2-F | H | 2 | H | H | 1,6 HCl · 1,2 $H_2O$ | 94—101 |
| 147 | $CH_3$ | H | 7-F | 8-$CH_3$ | H | H | 2 | H | H | Base | Ö |
| 148 | $CH_3$ | H | 7-$CH_3$ | 8-F | H | H | 2 | H | H | Base | Ö |
| 149 | $CH_3$ | H | 8-$CH_3$ | H | 2-F | H | 2 | H | H | Base · 0,1 $C_2H_5OH$ | 128—130 |
| 150 | $CH_3$ | H | 8-$OCH_3$ | H | 2-F | H | 2 | H | H | 1,65 HCl · 0,45 $H_2O$ | 112—116 |

Fortsetzung

| Beispiel Nr. | $R_1$ | $R_2$ | Substitution im Ring A $R_6$ | $R_7$ | Substitution im Ring B $R_8$ | $R_9$ | n | $R_4$ | $R_5$ | Salz | Fp °C |
|---|---|---|---|---|---|---|---|---|---|---|---|
| 151 | $CH_3$ | H | 8-$CH_3$ | H | 2-F | H | 2 | 4-$OCH_3$ | H | HCl · 0,39 $H_2O$ | 87— 93 |
| 152 | n-$C_4H_9$ | H | H | H | 2-F | H | 0 | 4-$CF_3$ | H | HCl · 0,3 i-$C_3H_7OH$ · 0,2 $H_2O$ | 128—131 |
| 153 | n-$C_4H_9$ | H | H | H | H | H | 0 | 4-$CF_3$ | H | HCl | 188—190 |
| 154 | $C_2H_5$ | H | H | H | 2-F | H | 0 | 4-$CF_3$ | H | p-tos · 0,1 $H_2O$ · 0,4 i-$C_3H_7OH$ | 170—172 |
| 155 | $C_2H_5$ | H | H | H | H | H | 0 | 4-$CF_3$ | H | p-tos | 210—212 |
| 156 | $C_2H_5$ | H | H | H | 2-F | H | 0 | 2-OH | H | cyclam | 155—157 |
| 157 | $C_2H_5$ | H | H | H | H | H | 0 | 2-OH | H | HCl · 0,05 i-$C_3H_7OH$ · 0,1 $H_2O$ | 183—185 |
| 158 | $CH_3$ | $(CH_3)_2CH$ | H | H | H | H | 0 | 2-Cl | H | Base | 152—154 |
| 159 | $CH_3$ | H | 7-F | H | H | H | 0 | 4-CN | H | Base | 166—167 |
| 160 | $CH_3$ | H | 7-F | H | 2-F | H | 0 | 4-CN | H | Base | 146—148 |
| 161 | $CH_3$ | H | 7-F | H | 2-F | H | 1 | 3,4-di-$OCH_3$ | | Base | 75— 81 |
| 162 | $CH_3$ | H | 8-$CH_3$ | H | 2-F | H | 1 | 3,4-di-$OCH_3$ | | Base | 118—121 |
| 163 | $CH_3$ | H | H | H | 2-Cl | H | 1 | 4-$OCH_3$ | H | HCl | 177—179 |
| 164 | $CH_3$ | H | 8-$CH_3$ | H | 4-$NO_2$ | H | 0 | 4-$OCH_3$ | H | HCl | 212—216 |
| 165 | $CH_3$ | H | 7-$CH_3$ | H | 4-F | H | 0 | 4-F | H | HCl | 225—227 |

Z = Zersetzung
HCl = Hydrochlorid
p-tos = p-Toluolsulfonate
Base = freie Base
Cyclan = Cyclamat
Ö = Öl

Beispiel I

Tabletten

Man stellt Tabletten mit folgender Zusammensetzung pro Tablette her:

| | |
|---|---|
| 1-Methyl-2-(3,4-dichlorbenzoylaminomethyl)-5-phenyl-1H-2,3-dihydro-1,4-benzodiazepin-hydrochlorid | 15 mg |
| Maisstärke | 60 mg |
| Milchzucker | 140 mg |
| Gelatine (10% Lösung) | 6 mg |

Der Wirkstoff, Maisstärke und der Milchzucker werden mit einer 10%igen Gelatine-Lösung eingedickt. Die Paste wird zerkleinert, das erhaltene Granulat wird auf ein geeignetes Blech gebracht und bei 45°C getrocknet. Das getrocknete Granulat wird durch eine Zerkleinerungsmaschine geleitet. Dann werden in einem Mixer die folgenden Substanzen zugemischt:

| | |
|---|---|
| Talkum | 5 mg |
| Magnesiumstearat | 5 mg |
| Maisstärke | 9 mg |

und die erhaltene Mischung sodann zu Tabletten von 240 mg verpreßt.

Beispiel II

Suppositorien

Man stellt Suppositorien mit folgender Zusammensetzung her:

| | |
|---|---|
| 1-Methyl-2-benzoylaminomethyl-5-phenyl-1H-2,3-dihydro-1,4-benzodiazepin | 15 mg |
| Kakaobutter | 1985 mg |

Der Wirkstoff und die fein geriebene Suppositoriengrundmasse werden gründlich gemischt und dann geschmolzen. Aus der durch Rühren homogen gehaltenen Schmelze werden Suppositorien von 2 Gramm gegossen.

Beispiel III

Injektionslösung

Eine parenterale Gebrauchsform wird mit den folgenden Bestandteilen hergestellt:

| | |
|---|---|
| 1-Methyl-2-benzoylaminomethyl-5-phenyl-1H-2,3-dihydro-1,4-benzodiazepin | 10% |
| Dimethylacetamid | 10% |
| Propylenglykol | 50% |
| Benzylalkohol | 1,5% |
| Äthanol | 10% |
| Wasser für Injektionszwecke | ad 100% |

Der Wirkstoff wird in Dimethylacetamid gelöst und mit Benzylalkohol, Propylenglykol, Äthanol und Wasser versetzt. Man filtriert durch einen Kerzenfilter, füllt in geeignete Ampullen, verschließt und sterilisiert.

**Patentansprüche für die Vertragsstaaten BE, CH, DE, FR, GB, IT, LI, LU, NL, SE**

1. 2-Phenylacylaminomethyl-1H-2,3-dihydro-1,4-benzodiazepine der Formel I

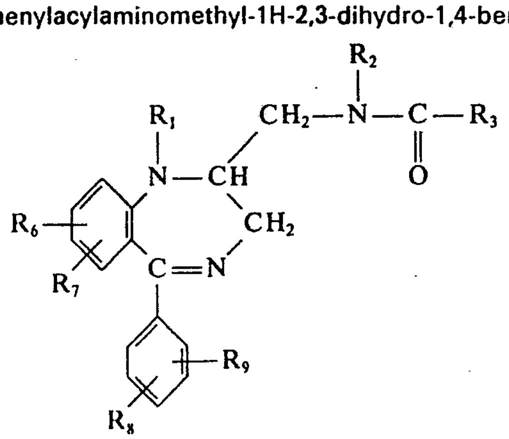

(I)

worin

$R_1$ Wasserstoff, Alkyl mit 1—4 Kohlenstoffatomen, Allyl, 2-Butenyl, 3-Butenyl oder Cyclopropylmethyl bedeutet,
$R_2$ Wasserstoff, Alkyl mit 1—4 Kohlenstoffatomen, Allyl, 2-Butenyl oder 3-Butenyl bedeutet.
$R_3$ eine Gruppe der Formel

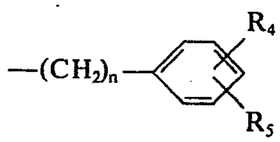

bedeutet, worin

$R_4$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituiertes Amino, Monoalkanoylamino mit 1—4 Kohlenstoffatomen oder Alkanoyloxy mit 1—4 Kohlenstoffatomen bedeutet, und
$R_5$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder
$R_4$ und $R_5$ an benachbarte Kohlenstoffatome gebunden sind und zusammen Methylendioxy oder Äthylendioxy bedeuten,
n 0,1 oder 2 bedeutet,
$R_6$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituiertes Amino, Monoalkanoylamino mit 1—4 Kohlenstoffatomen, oder Alkanoyloxy mit 1—4 Kohlenstoffatomen bedeutet, und
$R_7$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder
$R_6$ und $R_7$ an benachbarte Kohlenstoffatome gebunden sind und zusammen Methylendioxy oder Äthylendioxy bedeuten,
$R_8$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituierte Amino, Monoalkanoylamino mit 1—4 Kohlenstoffatomen oder Alkanoyloxy mit 1—4 Kohlenstoffatomen bedeutet, und
$R_9$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder
$R_8$ und $R_9$ an benachbarte Kohlenstoffatome gebunden sind und zusammen Methylendioxy oder Äthylendioxy bedeuten,

sowie deren optische Isomere und deren Säureadditionssalze.

2. 2-Phenylacylaminomethyl-1H-2,3-dihydro-1,4-benzodiazepine entsprechend Anspruch 1, in welchen $R_1$ Wasserstoff, Alkyl mit 1—4 Kohlenstoffatomen oder Cyclopropylmethyl ist.

3. 2-Phenylacylaminomethyl-1H-2,3-dihydro-1,4-benzodiazepine entsprechend den Ansprüchen 1 und 2, in welchen $R_2$ Wasserstoff ist.

4. 2-Phenylacylaminomethyl-1H-2,3-dihydro-1,4-benzodiazepine entsprechend Anspruch 1, worin

$R_1$ Alkyl mit 1—4 Kohlenstoffatomen bedeutet,
$R_2$ Wasserstoff bedeutet,
$R_4$ Wasserstoff, Halogen, Cyano, Alkyl mit 1—4 Kohlenstoffatomen oder Alkoxy mit 1—4 Kohlenstoffatomen bedeutet,
$R_5$ Wasserstoff, Halogen, Alkyl mit 1—4 Kohlenstoffatomen oder Alkoxy mit 1—4 Kohlenstoffatomen bedeutet,
n 0,1 oder 2 bedeutet,
$R_6$ Wasserstoff, Halogen, Alkyl mit 1—4 Kohlenstoffatomen. Alkoxy mit 1—4 Kohlenstoffatomen oder Nitro bedeutet, und
$R_7$ Wasserstoff, Halogen, Alkyl mit 1—4 Kohlenstoffatomen oder Alkoxy mit 1—4 Kohlenstoffatomen bedeutet, oder
$R_6$ und $R_7$ an benachbarte Kohlenstoffatome gebunden sind und zusammen Methylendioxy oder Äthylendioxy bedeuten,
$R_8$ Wasserstoff, Halogen, Alkyl mit 1—4 Kohlenstoffatomen, Alkoxy mit 1—4 Kohlenstoffatomen oder Trifluormethyl bedeutet, und
$R_9$ Wasserstoff, Halogen, Alkyl mit 1—4 Kohlenstoffatomen oder Alkoxy mit 1—4 Kohlenstoffatomen bedeutet.

5. 2-Methyl-2-(4-cyanobenzoylaminomethyl)-5-(2-fluorphenyl)-1H-2,3-dihydro-1,4-benzodiazepin und dessen Säureadditionssalze gemäß Anspruch 1.

6. 1-Methyl-2-benzoylaminomethyl-5-phenyl-1H-2,3-dihydro-1,4-benzodiazepin und dessen Säureadditionssalze gemäß Anspruch 1.

7. 1-Methyl-8-fluor-2-benzoylaminomethyl-5-phenyl-1H-2,3-dihydro-1,4-benzodiazepin und dessen Säureadditionssalze gemäß Anspruch 1.

8. 1,8-Dimethyl-2-(4-methylbenzoylaminomethyl)-5-(4-fluorphenyl)-1H-2,3-dihydro-1,4-benzodiazepin und dessen Säureadditionssalze gemäß Anspruch 1.

9. Verfahren zur Herstellung von 2-Phenylacylaminomethyl-1H-2,3-dihydro-1,4-benzodiazepinen der Formel I

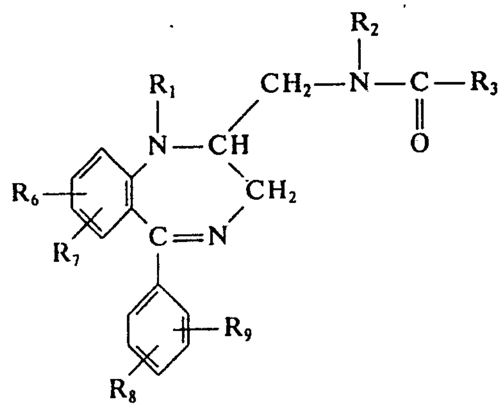

(I)

worin $R_1$, $R_2$, $R_3$, $R_6$, $R_7$, $R_8$ und $R_9$ die in Anspruch I angegebene Bedeutung besitzen, sowie deren optischen Isomeren und deren Säureadditionssalzen, dadurch gekennzeichnet, daß man 2-Aminomethyl-1H-2,3-dihydro-1,4-benzodiazepine der Formel II

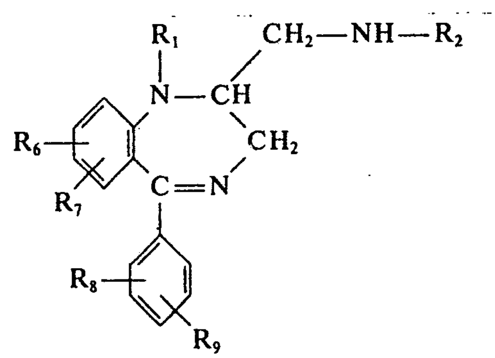

(II)

worin $R_1$, $R_2$, $R_6$, $R_7$, $R_8$ und $R_9$ die oben angegebenen Bedeutungen haben, oder deren Säureadditionssalze mit einer Carbonsäure oder einem reaktiven Carbonsäurederivat der Formel III,

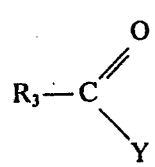

(III)

worin $R_3$ die obengenannte Bedeutung hat und Y Hydroxy, Halogen, Alkoxy mit 1—4 Kohlenstoffatomen oder eine O—CO—Z-Gruppe bedeutet, worin Z die Bedeutung $R_3$ oder Alkoxy mit 1—4 Kohlenstoffatomen hat, in einem inerten Lösungsmittel bei Temperaturen zwischen —30°C und dem Siedepunkt des eingesetzten Lösungsmittels acyliert und gegebenenfalls Verbindungen der Formel I, worin $R_2$ Wasserstoff bedeutet, zu Verbindungen der Formel I, worin $R_2$ Alkyl mit 1—4 Kohlenstoffatomen, Allyl, 2-Butenyl oder 3-Butenyl bedeutet, alkyliert und/oder in Verbindungen der Formel I, worin $R_6$ und/oder $R_7$ Wasserstoff bedeuten, in den Phenylring Chlor, Brom, Hydroxy oder Nitro einführt, und/oder in Verbindungen der Formel I, worin $R_4$, $R_5$, $R_6$, $R_7$, $R_8$ und/oder $R_9$ Alkanoyloxy mit 1—4 Kohlenstoffatomen oder Alkanoylamino mit 1—4 Kohlenstoffatomen bedeuten, diese Gruppen zu Hydroxy oder Amino hydrolysiert, und gegebenenfalls racemische Gemische von Verbindungen der Formel I in ihre optischen Isomeren auftrennt und gegebenenfalls freie Verbindungen der Formel I in

ihre Säureadditionssalze überführt oder die Säureadditionssalze in die freien Verbindungen der Formel I überführt.

10. Arzneimittel, enthaltend eine Verbindung gemäß Anspruch I und übliche pharmazeutische Trägerstoffe.

**Patentansprüche für den Vertragsstaat AT**

1. Verfahren zur Herstellung von 2-Phenylacylaminomethyl-1H-2,3-dihydro-1,4-benzodiazepinen der Formel I

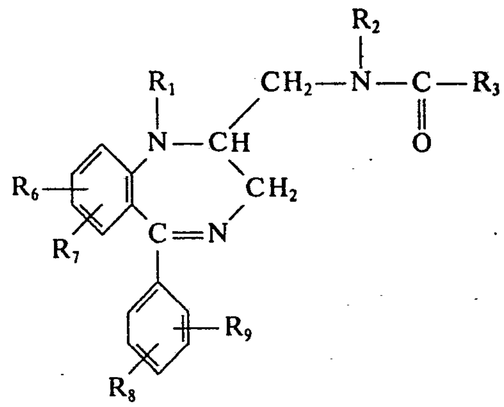

(I)

worin

$R_1$ Wasserstoff, Alkyl mit 1—4 Kohlenstoffatomen, Allyl, 2-Butenyl, 3-Butenyl oder Cyclopropylmethyl bedeutet,
$R_2$ Wasserstoff, Allyl mit 1—4 Kohlenstoffatomen, Allyl, 2-Butenyl oder 3-Butenyl bedeutet.
$R_3$ eine Gruppe der Formel

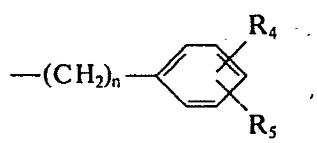

bedeutet, worin

$R_4$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituiertes Amino, Monoalkanoylamino mit 1—4 Kohlenstoffatomen oder Alkanoyloxy mit 1—4 Kohlenstoffatomen bedeutet, und
$R_5$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder
$R_4$ und $R_5$ an benachbarte Kohlenstoffatome gebunden sind und zusammen Methylendioxy oder Äthylendioxy bedeuten,
n 0,1 oder 2 bedeutet,
$R_6$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituiertes Amino, Monoalkanoylamino mit 1—4 Kohlenstoffatomen, oder Alkanoyloxy mit 1—4 Kohlenstoffatomen bedeutet, und
$R_7$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder
$R_6$ und $R_7$ an benachbarte Kohlenstoffatome gebunden sind und zusammen Methylendioxy oder Äthylendioxy bedeuten,
$R_8$ Wasserstoff, Halogen, Alkyl mit 1—4 Kohlenstoffatomen, Alkoxy mit 1—4 Kohlenstoffatomen, Hydroxy, Alkylthio mit 1—4 Kohlenstoffatomen, Nitro, Trifluormethyl, Cyano, Amino, durch Alkyl mit 1—4 Kohlenstoffatomen mono- oder disubstituierte Amino, Monoalkanoylamino mit 1—4 Kohlenstoffatomen oder Alkanoyloxy mit 1—4 Kohlenstoffatomen bedeutet, und
$R_9$ Wasserstoff, Halogen, Alkyl mit 1—4 Kohlenstoffatomen, Alkoxy mit 1—4 Kohlenstoffatomen, Hydroxy oder Alkanoyloxy mit 1—4 Kohlenstoffatomen bedeutet, oder
$R_8$ und $R_9$ an benachbarte Kohlenstoffatome gebunden sind und zusammen Methylendioxy oder Äthy lendioxy bedeuten,

sowie deren Isomeren und deren Säureadditionssalzen, dadurch gekennzeichnet, daß man 2-Aminomethyl-1H-2,3-dihydro-1,4-benzodiazepine der Formel II

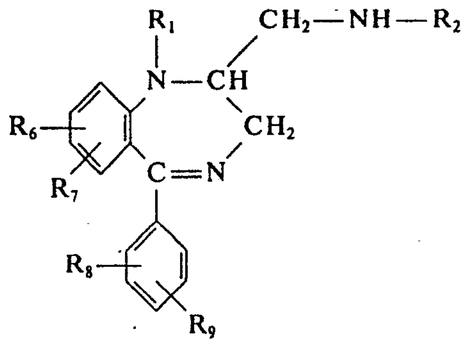

(II)

worin $R_1$, $R_2$, $R_6$, $R_7$, $R_8$ und $R_9$ die oben angegebenen Bedeutungen haben, oder deren Säureadditionssalze mit einer Carbonsäure oder einem reaktiven Carbonsäurederivat der Formel III,

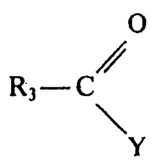

(III)

worin $R_3$ die obengenannte Bedeutung hat und Y Hydroxy, Halogen, Alkoxy mit 1—4 Kohlenstoffatomen oder eine O—CO—Z-Gruppe bedeutet, worin z die Bedeutung $R_3$ oder Alkoxy mit 1—4 Kohlenstoffatomen hat, in einem inerten Lösungsmittel bei Temperaturen zwischen —30°C und dem Siedepunkt des eingesetzten Lösungsmittels acyliert und gegebenenfalls Verbindungen der Formel I, worin $R_2$ Wasserstoff bedeutet, zu Verbindungen der Formel I, worin $R_2$ Alkyl mit 1—4 Kohlenstoffatomen, Allyl, 2-Butenyl oder 3-Butenyl bedeutet, alkyliert und/oder in Verbindungen der Formel I, worin $R_6$ und/oder $R_7$ Wasserstoff bedeuten, in den Phenylring Chlor, Brom, Hydroxy oder Nitro einführt, und/oder in Verbindungen der Formel I, worin $R_4$, $R_5$, $R_6$, $R_7$, $R_8$ und/oder $R_9$ Alkanoyloxy mit 1—4 Kohlenstoffatomen oder Alkanoylamino mit 1—4 Kohlenstoffatomen bedeuten, diese Gruppen zu Hydroxy oder Amino hydrolysiert, und gegebenenfalls racemische Gemische von Verbindungen der Formel I in ihre optischen Isomeren auftrennt und gegebenenfalls freie Verbindungen der Formel I in ihre Säureadditionssalze überführt oder die Säureadditionssalze in die freien Verbindungen der Formel I überführt.

2. Verfahren nach Anspruch 1, dadurch gekennzeichnet, daß man 2-Phenylacylaminomethyl-1H-2,3-dihydro-1,4-benzodiazepine herstellt, in welchen $R_1$ Wasserstoff, Alkyl mit 1—4 Kohlenstoffatomen oder Cyclopropylmethyl ist.

3. Verfahren nach Anspruch 1 oder 2, dadurch gekennzeichnet, daß man 2-Phenylacylaminomethyl-1H-2,3-dihydro-1,4-benzodiazepine herstellt, in welchen $R_2$ Wasserstoff ist.

4. Verfahren nach Anspruch 1, dadurch gekennzeichnet, daß man 2-Phenylacylaminomethyl-1H-2,3-dihydro-1,4-benzodiazepine herstellt, worin

$R_1$ Alkyl mit 1—4 Kohlenstoffatomen bedeutet,
$R_2$ Wasserstoff bedeutet,
$R_4$ Wasserstoff, Halogen, Cyano, Alkyl mit 1—4 Kohlenstoffatomen oder Alkoxy mit 1—4 Kohlenstoffatomen bedeutet,
$R_5$ Wasserstoff, Halogen, Alkyl mit 1—4 Kohlenstoffatomen oder Alkoxy mit 1—4 Kohlenstoffatomen bedeutet,
n 0,1 oder 2 bedeutet,
$R_6$ Wasserstoff, Halogen, Alkyl mit 1—4 Kohlenstoffatomen oder Alkoxy mit 1—4 Kohlenstoffatomen bedeutet, und
$R_7$ Wasserstoff, Halogen, Alkyl mit 1—4 Kohlenstoffatomen oder Alkoxy mit 1—4 Kohlenstoffatomen bedeutet, oder
$R_6$ und $R_7$ an benachbarte Kohlenstoffatome gebunden sind und zusammen Methylendioxy oder Äthylendioxy bedeuten,
$R_8$ Wasserstoff, Halogen, Alkyl mit 1—4 Kohlenstoffatomen, Alkoxy mit 1—4 Kohlenstoffatomen oder Trifluormethyl bedeutet, und
$R_9$ Wasserstoff, Halogen, Alkyl mit 1—4 Kohlenstoffatomen oder Alkoxy mit 1—4 Kohlenstoffatomen bedeutet.

5. Verfahren nach Anspruch 1, dadurch gekennzeichnet, daß man 2-Methyl-2-(4-cyanobenzoylaminomethyl)-5-(2-fluorphenyl)-1H-2,3-dihydro-1,4-benzodiazepin und dessen Säureadditionssalze herstellt.

6. Verfahren nach Anspruch 1, dadurch gekennzeichnet, daß man 1-Methyl-2-benzoylaminomethyl-5-phenyl-1H-2,3-dihydro-1,4-benzodiazepin und dessen Säureadditionssalze herstellt.

7. Verfahren nach Anspruch 1, dadurch gekennzeichnet, daß man 1-Methyl-8-fluor-2-benzoylaminomethyl-5-phenyl-1H-2,3-dihydro-1,4-benzodiazepin und dessen Säureadditionssalze herstellt.

8. Verfahren nach Anspruch 1, dadurch gekennzeichnet, daß man 1,8-Dimethyl-2-(4-methylbenzoylaminomethyl)-5-(4-fluorphenyl)-1H-2,3-dihydro-1,4-benzodiazepin und dessen Säureadditionssalze herstellt.

**Claims for the Contracting States, BE, CH, DE, FR, GB, IT, LI, LU, NL, SE**

1. 2-Phenylacylaminomethyl-1H-2,3-dihydro-1,4-benzodiazepines of the formula I

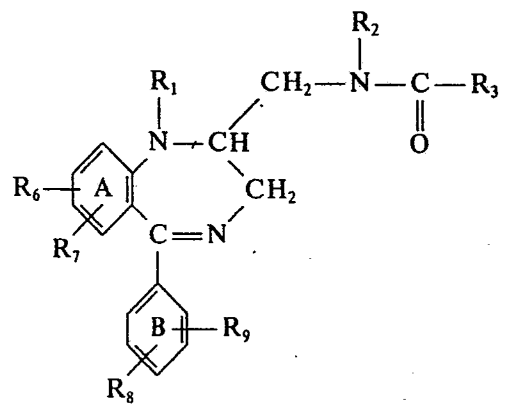

(I)

in which

$R_1$ is hydrogen, alkyl with 1 to 4 carbon atoms, allyl, 2-butenyl, 3-butenyl or cyclopropylmethyl,
$R_2$ is hydrogen, alkyl with 1 to 4 carbon atoms, allyl, 2-butenyl or 3-butenyl,
$R_3$ is a group of the formula

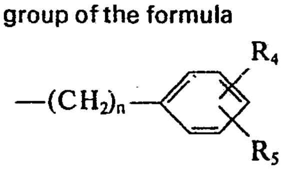

in which

$R_4$ is hydrogen, halogen, alkyl with 1 to 4 carbon atoms, alkoxy with 1 to 4 carbon atoms, hydroxy, alkylthio with 1 to 4 carbon atoms, nitro, trifluoromethyl, cyano, amino, amino mono- or di-substituted by alkyl with 1 to 4 carbon atoms monoalkanoylamino with 1 to 4 carbon atoms or alkanoyloxy with 1 to 4 carbon atoms, and
$R_5$ is hydrogen, halogen, alkyl with 1 to 4 carbon atoms, alkoxy with 1 to 4 carbon atoms, hydroxy or alkanoyloxy with 1 to 4 carbon atoms, or
$R_4$ and $R_5$ are bonded to adjacent carbon atoms and together are methylene dioxy or ethylene dioxy,
n is 0,1 or 2,
$R_6$ is hydrogen, halogen, alkyl with 1 to 4 carbon atoms, alkoxy with 1 to 4 carbon atoms, hydroxy, alkylthio with 1 to 4 carbon atoms, nitro, trifluoromethyl, cyano, amino, amino mono- or di-substituted by alkyl with 1 to 4 carbon atoms, monoalkanoylamino with 1 to 4 carbon atoms, alkanoyloxy with 1 to 4 carbon atoms, and
$R_7$ is hydrogen, halogen, alkyl with 1 to 4 carbon atoms, alkoxy with 1 to 4 carbon atoms, or
$R_6$ and $R_7$ are bonded to adjacent carbon atoms and together are methylene dioxy or ethylene dioxy,
$R_8$ is hydrogen, halogen, alkyl with 1 to 4 carbon atoms, alkoxy with 1 to 4 carbon atoms, hydroxy, alkylthio with 1 to 4 carbon atoms, nitro, trifluoromethyl, cyano, amino, amino mono- or disubstituted by alkyl with 1 to 4 carbon atoms monoalkanoylamino with 1 to 4 carbon atoms or alkanoyloxy with 1 to 4 carbon atoms, and
$R_9$ is hydrogen, halogen, alkyl with 1 to 4 carbon atoms, alkoxy with 1 to 4 carbon atoms, hydroxy or alkanoyloxy with 1 to 4 carbon atoms, or
$R_8$ and $R_9$ are bonded to adjacent carbon atoms and together signify methylene dioxy or ethylene dioxy,

and their optical isomers and their acid addition salts.

2. 2-Phenylacylaminomethyl-1H-2,3-dihydro-1,4-benzodiazepines according to Claim 1, in which $R_1$ is hydrogen, alkyl with 1 to 4 carbon atoms or cyclopropylmethyl.

3. 2-Phenylacylaminomethyl-1H-2,3-dihydro-1,4-benzodiazepines according to Claims 1 and 2, in which $R_2$ is hydrogen.

4. 2-Phenylacylaminomethyl-1H-2,3-dihydro-1,4-benzodiazepines according to Claim 1 in which

$R_1$ is alkyl with 1 to 4 carbon atoms,
$R_2$ is hydrogen,
$R_4$ is hydrogen, halogen, cyano, alkyl with 1 to 4 carbon atoms or alkoxy with 1 to 4 carbon atoms,
$R_5$ is hydrogen, halogen, alkyl with 1 to 4 carbon atoms or alkoxy with 1 to 4 carbon atoms,
n is 0,1 or 2,
$R_6$ is hydrogen, halogen, alkyl with 1 to 4 carbon atoms, alkoxy with 1 to 4 carbon atoms or nitro, and,
$R_7$ is hydrogen, halogen, alkyl with 1 to 4 carbon atoms or alkoxy with 1 to 4 carbon atoms, or
$R_6$ and $R_7$ are bonded to adjacent carbon atoms and together are methylene dioxy or ethylene dioxy,
$R_8$ is hydrogen, halogen, alkyl with 1 to 4 carbon atoms, alkoxy with 1 to 4 carbon atoms or trifluoromethyl, and
$R_9$ is hydrogen, halogen, alkyl with 1 to 4 carbon atoms or alkoxy with 1 to 4 carbon atoms.

5. 2-Methyl-2-(4-cyanobenzoylaminomethyl)-5-(2-fluorophenyl)-1H-2,3-dihydro-1,4-benzodiazepine and its acid addition salts according to Claim 1.

6. 1-Methyl-2-benzoylaminomethyl-5-phenyl-1H-2,3-dihydro-1,4-benzodiazepine and its acid addition salts according to Claim 1.

7. 1-Methyl-8-fluoro-2-benzoylaminomethyl-5-phenyl-1H-2,3-dihydro-1,4-benzodiazepine and its acid addition salts according to Claim 1.

8. 1,8-Dimethyl-2-(4-methylbenzoylaminomethyl)-5-(4-fluorophenyl)-1H-2,3-dihydro-1,4-benzodiazepine and its acid addition salts according to Claim 1.

9. Process for the preparation of 2-phenylacylaminomethyl-1H-2,3-dihydro-1,4-benzodiazepines of formula I

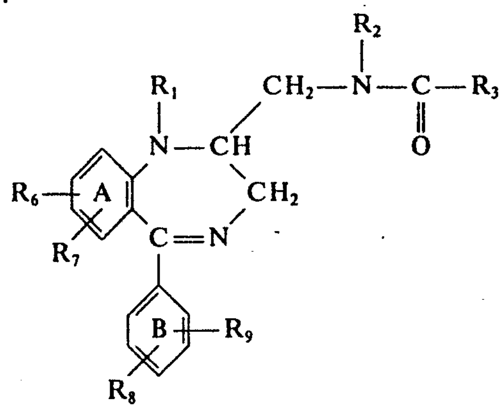

(I)

in which $R_1$, $R_2$, $R_3$, $R_6$, $R_7$, $R_8$ and $R_9$ have the meanings given in Claim 1, and their optical isomers and their acid addition salts, characterized in that 2-aminomethyl-1H-2,3-dihydro-1,4-benzodiazepines of formula II

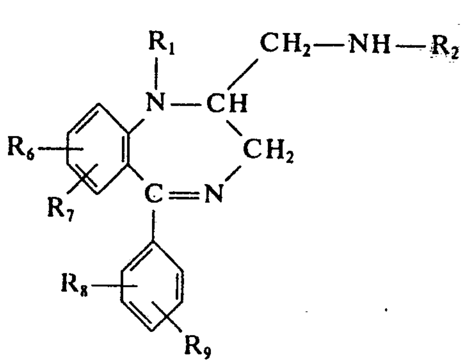

(II)

in which $R_1$, $R_2$, $R_6$, $R_7$, $R_8$ and $R_9$ have the meanings given above, or their acid addition salts with a carboxylic acid or a reactive carboxylic acid derivative of formula III,

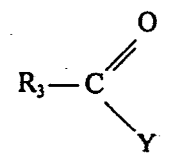

(III)

in which $R_3$ has the above mentioned meaning and Y is hydroxy, halogen, alkoxy with 1 to 4 carbon atoms or an O—CO—Z-group, in which Z has the meaning $R_3$ or alkoxy with 1 to 4 carbon atoms, are acylated in an inert solvent at temperatures between −30° C., and the boiling point of the solvent used and if required compounds of formula I, in which $R_2$ is hydrogen, are alkylated to compounds of formula I, in which $R_2$ is alkyl with 1 to 4 carbon atoms, allyl, 2-butenyl or 3-butenyl, and/or in compounds of formula I, in which $R_6$ and/or $R_7$ is/are hydrogen, there is introduced into the phenyl ring chlorine, bromine, hydroxy or nitro, and/or in compounds of formula I, in which $R_4$, $R_5$, $R_6$, $R_7$, $R_8$ and/or $R_9$ is/are alkanoyloxy with 1 to 4 carbon atoms or alkanoylamino with 1 to 4 carbon atoms, these groups are hydrolysed to hydroxy or amino, and if required racemic mixtures of compounds of formula I are separated into their optical isomers and if required free compounds of formula I are converted into their acid addition salts or the acid addition salts are converted into the free compounds of formula I.

10. Medicaments containing a compound according to Claim 1 and conventional pharmaceutical carrier substances.

**Claims for the Contracting State AT**

1. Process for the preparation of 2-phenyl-acylaminomethyl-1H-2,3-dihydro-1,4-benzodiazepines of the formula I

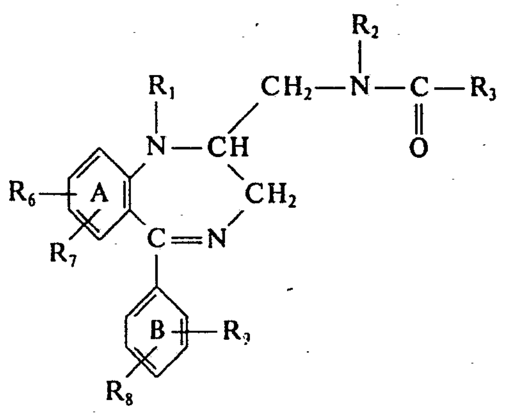

(I)

in which

$R_1$ is hydrogen, alkyl with 1 to 4 carbon atoms, allyl, 2-butenyl, 3-butenyl or cyclopropylmethyl,
$R_2$ is hydrogen, alkyl with 1 to 4 carbon atoms, allyl, 2-butenyl or 3-butenyl,
$R_3$ is a group of the formula

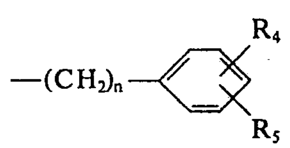

in which

$R_4$ is hydrogen, halogen, alkyl with 1 to 4 carbon atoms, alkoxy with 1 to 4 carbon atoms, hydroxy, alkylthio with 1 to 4 carbon atoms, nitro, trifluoromethyl, cyano, amino, amino mono- or di-substituted by alkyl with 1 to 4 carbon atoms monoalkanoylamino with 1 to 4 carbon atoms or alkanoyloxy with 1 to 4 carbon atoms, and
$R_5$ is hydrogen, halogen, alkyl with 1 to 4 carbon atoms, alkoxy with 1 to 4 carbon atoms, hydroxy or alkanoyloxy with 1 to 4 carbon atoms, or
$R_4$ and $R_5$ are bonded to adjacent carbon atoms and together are methylene dioxy or ethylene dioxy,
n is 0,1 or 2,
$R_6$ is hydrogen, halogen, alkyl with 1 to 4 carbon atoms, alkoxy with 1 to 4 carbon atoms, hydroxy, alkylthio with 1 to 4 carbon atoms, nitro, trifluoromethyl, cyano, amino, amino mono- or di-substituted by alkyl with 1 to 4 carbon atoms, monoalkanoylamino with 1 to 4 carbon atoms, alkanoyloxy with 1 to 4 carbon atoms, and

$R_7$ is hydrogen, halogen, alkyl with 1 to 4 carbon atoms, alkoxy with 1 to 4 carbon atoms, or
$R_6$ and $R_7$ are bonded to adjacent carbon atoms and together are methylene dioxy or ethylene dioxy,
$R_8$ is hydrogen, halogen, alkyl with 1 to 4 carbon atoms, alkoxy with 1 to 4 carbon atoms, hydroxy, alkylthio with 1 to 4 carbon atoms, nitro, trifluoromethyl, cyano, amino, amino mono- or disubstituted by alkyl with 1 to 4 carbon atoms monoalkanoylamino with 1 to 4 carbon atoms or alkanoyloxy with 1 to 4 carbon atoms, and
$R_9$ is hydrogen, halogen, alkyl with 1 to 4 carbon atoms, alkoxy with 1 to 4 carbon atoms, hydroxy or alkanoyloxy with 1 to 4 carbon atoms, or
$R_8$ and $R_9$ are bonded to adjacent carbon atoms and together signify methylene dioxy or ethylene dioxy,

and their isomers and their acid addition salts, characterized in that 2-aminomethyl-1H-2,3-dihydro-1,4-benzodiazepines of formula II

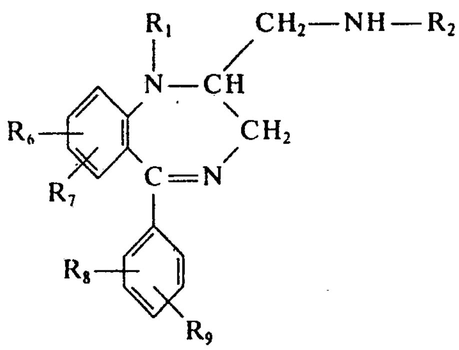

(II)

in which $R_1$, $R_2$, $R_6$, $R_7$, $R_8$ and $R_9$ have the meanings given above, or their acid addition salts with a carboxylic acid or a reactive carboxylic acid derivative of formula III,

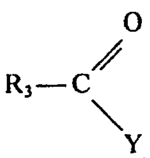

(III)

in which $R_3$ has the above mentioned meaning and Y is hydroxy, halogen, alkoxy with 1 to 4 carbon atoms or an O—CO—Z-group, in which Z has the meaning $R_3$ or alkoxy with 1 to 4 carbon atoms, are acylated in an inert solvent at temperatures between −30° C., and the boiling point of the solvent used and if required compounds of formula I, in which $R_2$ is hydrogen, are alkylated to compounds of formula I, in which $R_2$ is alkyl with 1 to 4 carbon atoms, allyl, 2-butenyl or 3-butenyl, and/or in compounds of formula I, in which $R_6$ and/or $R_7$ is/are hydrogen, there is introduced into the phenyl ring chlorine, bromine, hydroxy or nitro, and/or in compounds of formula I, in which $R_4$, $R_5$, $R_6$, $R_7$, $R_8$ and/or $R_9$ is/are alkanoyloxy with 1 to 4 carbon atoms or alkanoylamino with 1 to 4 carbon atoms, these groups are hydrolysed to hydroxy or amino, and if required racemic mixtures of compounds of formula I are separated into their optical isomere and if required free compounds of formula I are converted into their acid addition salts or the acid addition salts are converted into the free compounds of formula I.

2. Process according to Claims 1, characterised in that 2-phenylacylaminomethyl-1H-2,3-dihydro-1,4-benzodiazepines are prepared, in which $R_1$ is hydrogen, alkyl with 1 to 4 carbon atoms or cyclopropylmethyl.

3. Process according to Claim 1 or 2, characterized in that 2-phenylacylaminomethyl-1H-2,3-dihydro-1,4-benzodiazepines are prepared, in which $R_2$ is hydrogen.

4. Process according to Claim 1, characterized in that 2-phenylacylaminomethyl-1H-2,3-dihydro-1,4-benzodiazepines are prepared in which

$R_1$ is alkyl with 1 to 4 carbon atoms,
$R_2$ is hydrogen,
$R_4$ is hydrogen, halogen, cyano, alkyl with 1 to 4 carbon atoms or alkoxy with 1 to 4 carbon atoms,
$R_5$ is hydrogen, halogen, alkyl with 1 to 4 carbon atoms or alkoxy with 1 to 4 carbon atoms,
n is 0,1 or 2,
$R_6$ is hydrogen, halogen, alkyl with 1 to 4 carbon atoms, alkoxy with 1 to 4 carbon atoms or nitro, and,
$R_7$ is hydrogen, halogen, alkyl with 1 to 4 carbon atoms or alkoxy with 1 to 4 carbon atoms, or
$R_6$ and $R_7$ are bonded to adjacent carbon atoms and together are methylene dioxy or ethylene dioxy,

$R_8$ is hydrogen, halogen, alkyl with 1 to 4 carbon atoms, alkoxy with 1 to 4 carbon atoms or trifluoromethyl, and

$R_9$ is hydrogen, halogen, alkyl with 1 to 4 carbon atoms or alkoxy with 1 to 4 carbon atoms.

5. Process according to Claim 1, characterized in that 2-methyl-2-(4-cyanobenzoylaminomethyl)-5-(2-fluorophenyl)-1H-2,3-dihydro-1,4-benzodiazepine and its acid addition salts are prepared.

6. Process according to Claim 1, characterized in that 1-methyl-2-benzoylaminomethyl-5-phenyl-1H-2,3-dihydro-1,4-benzodiazepine and its acid addition salts are prepared.

7. Process according to Claim 1, characterized in that 1-methyl-8-fluoro-2-benzoylaminomethyl-5-phenyl-1H-2,3-dihydro-1,4-benzodiazepine and its acid addition salts are prepared.

8. Process according to Claim 1, characterized in that 1,8-dimethyl-2-(4-methylbenzoylaminomethyl)-5-(4-fluorophenyl)-1H-2,3-dihydro-1,4-benzodiazepine and its acid addition salts are prepared.

**Revendications pour les Etats contractants: BE, CH, DE, FR, GB, IT, LI, LU, NL, SE**

1. 2-Phénylacylaminométhyl-1H-2,3-dihydro-1,4-benzodiazépines de formule I

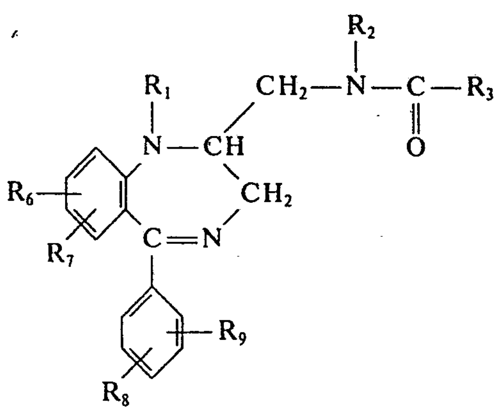

dans laquelle:

$R_1$ représente un atome d'hydrogène, un groupe alkyle à 1 à 4 atomes de carbone, allyle, 2-butényle, 3-butényle ou cyclopropylméthyle,

$R_2$ représente un atome d'hydrogène, un groupe alkyle à 1 à 4 atomes de carbone, allyle, 2-butényle ou 3-butényle

$R_3$ represente un groupe de formule

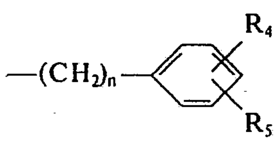

dans laquelle:

$R_4$ représente un atome d'hydrogène, un atome d'halogène, un groupe alkyle à 1 à 4 atomes de carbone, alcoxy à 1 à 4 atomes de carbone, hydroxy, alkylthio à 1 à 4 atomes de carbone, nitro, trifluorométhyle, cyano, amino, amino mono- ou disubstitué par un groupe alkyle à 1 à 4 atomes de carbone, monoalcanoylamino à 1 à 4 atomes de carbone ou alcanoyloxy à 1 à 4 atomes de carbone et

$R_5$ représente un atome d'hydrogène, un atome d'halogène, un groupe alkyle à 1 à 4 atomes de carbone, alcoxy à 1 à 4 atomes de carbone, hydroxy ou alcanoyloxy à 1 à 4 atomes de carbone ou bien

$R_4$ et $R_5$ sont liés à des atomes de carbone voisins et forment ensemble un groupe méthylènedioxy ou éthylènedioxy,

n est égal à 0,1 ou 2,

$R_6$ représente un atome d'hydrogène, un atome d'halogène, un groupe alkyle à 1 à 4 atomes de carbone, alcoxy à 1 à 4 atomes de carbone, hydroxy, alkylthio à 1 à 4 atomes de carbone, nitro, trifluorométhyle, cyano, amino, amino mono ou disubstitué par un groupe alkyle à 1 à 4 atomes de carbone, monoalcanoylamino à 1 à 4 atomes de carbone ou alcanoyloxy à 1 à 4 atomes de carbone et

$R_7$ représente un atome d'hydrogène, un atome d'halogène, un groupe alkyle à 1 à 4 atomes de

carbone, alcoxy à 1 à 4 atomes de carbone, hydroxy ou alcanoyloxy à 1 à 4 atomes de carbone, ou bièn

$R_6$ et $R_7$ sont liés à des atomes de carbone voisins et forment ensemble un groupe méthylènedioxy ou éthylènedioxy,

$R_8$ représente un atome d'hydrogène, un atome d'halogène, un groupe alkyle à 1 à 4 atomes de carbone, alcoxy à 1 à 4 atomes de carbone, hydroxy, alkylthio à 1 à 4 atomes de carbone, nitro, trifluorométhyle, cyano, amino, amino mono- ou disubstitué par un groupe alkyle à 1 à 4 atomes de carbone, monoalcanoylamino à 1 à 4 atomes de carbone ou alcanoyloxy à 1 à 4 atomes de carbone, et

$R_9$ représente un atome d'hydrogène, un atome d'halogène, un groupe alkyle à 1 à 4 atomes de carbone, alcoxy à 1 à 4 atomes de carbone, hydroxy ou alcanoyloxy à 1 à 4 atomes de carbone ou bien

$R_8$ et $R_9$ sont liés à des atomes de carbone voisins et forment ensemble un groupe méthylènedioxy ou éthylènedioxy,

ainsi que leurs isomères optiques et leurs sels d'addition d'acides.

2. 2-phénylacylaminométhyl-1H-2,3-dihydro-1,4-benzodiazépines selon la revendication 1, dans lesquelles $R_1$ est un atome d'hydrogène, un groupe alkyle à 1 à 4 atomes de carbone ou un groupe cyclopropylméthyle.

3. 2-phénylacylaminométhyl-1H-2,3-dihydro-1,4-benzodiazépines selon les revendications 1 et 2, dans lesquelles $R_2$ est l'hydrogène.

4. 2-phénylacylaminométhyl-1H-2,3-dihydro-1,4-benzodiazépines selon la revendication 1, dans lesquelles

$R_1$ représente un groupe alkyle à 1 à 4 atomes de carbone,

$R_2$ représente un atome d'hydrogéne

$R_4$ représente un atome d'hydrogène, un atome d'halogène, un groupe cyano, un groupe alkyle à 1 à 4 atomes de carbone ou alcoxy à 1 à 4 atomes de carbone,

$R_5$ représente un atome d'hydrogène, un atome d'halogène, un groupe alkyle à 1 à 4 atomes de carbone ou alcoxy à 1 à 4 atomes de carbone,

n est égal à 0,1 ou 2,

$R_6$ représente un atome d'hydrogène, un atome d'halogène, un groupe alkyle à 1 à 4 atomes de carbone, alcoxy à 1 à 4 atomes de carbone ou un groupe nitro et

$R_7$ représente un atome d'hydrogène, un atome d'halogène, un groupe alkyle à 1 à 4 atomes de carbone ou alcoxy à 1 à 4 atomes de carbone, ou bien

$R_6$ et $R_7$ sont liés à des atomes de carbone voisins et forment ensemble un groupe méthylènedioxy ou éthylènedioxy.

$R_8$ représente un atome d'hydrogène, un atome d'halogène, un groupe alkyle à 1 à 4 atomes de carbone, alcoxy à 1 à 4 atomes de carbone ou trifluorométhyle et

$R_9$ représente un atome d'hydrogène, un atome d'halogène, un groupe alkyle à 1 à 4 atomes de carbone ou alcoxy à 1 à 4 atomes de carbone.

5. 2-méthyl-2-(4-cyanobenzoylaminométhyl)-5-(2-fluorophényl)-1H-2,3-dihydro-1,4-benzodiazépine et ses sels d'addition d'acides selon la revendication 1.

6. 1-méthyl-2-benzoylaminométhyl-5-phényle-1H-2,3-dihydro-1,4-benzodiazépine et ses sels d'addition d'acides selon la revendication 1.

7. 1-méthyl-8-fluoro-2-benzoylaminométhyl-5-phényl-1H,2,3-dihydro-1,4-benzodiazépine et ses sels d'addition d'acides selon la revendication 1.

8. 1,8-diméthyl-2-(4-méthylbenzoylaminométhyl)-5-(4-fluorophényl)-1H-2,3-dihydro-1,4-benzodiazépine et ses sels d'addition d'acides selon la revendication 1.

9. Procédé de préparation de 2-Phénylacylaminométhyl-1H-2,3-dihydro-1,4-benzodiazépines de formule I

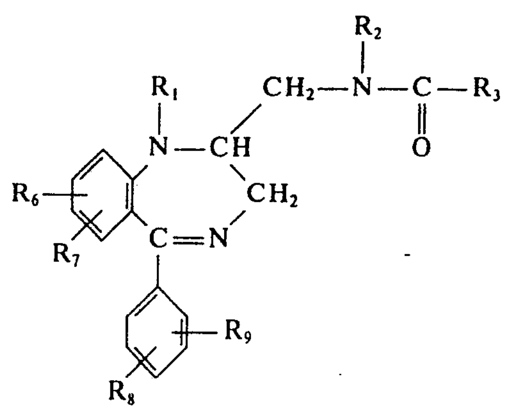

dans laquelle $R_1$, $R_2$, $R_3$, $R_6$, $R_7$, $R_8$ et $R_9$ ont la signification indiquée dans la revendication 1, ainsi que de leurs isomères optiques et de leurs sels d'addition d'acides, caractérisé en ce que l'on acyle des 2-aminométhyl-1H-2,3-dihydro-1,4-benzodiazépines de formule II

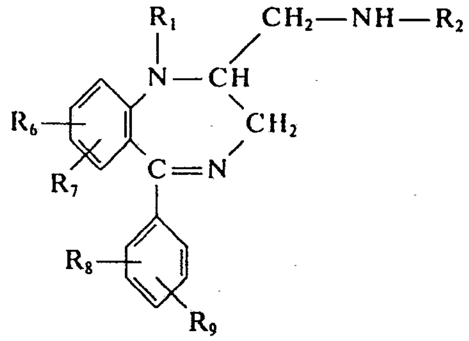

(II)

dans laquelle $R_1$, $R_2$, $R_6$, $R_7$, $R_8$ et $R_9$ ont les significations indiquées ci-dessus ou leurs sels d'addition d'acides avec un acide carboxylique ou un dérivé réactif d'un acide carboxylique de formule III

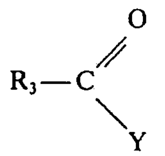

(III)

dans laquelle $R_3$ a la signification indiquée ci-dessus et Y représente un groupe hydroxy, un atome d'halogène, un groupe alcoxy à 1 à 4 atomes de carbone ou un groupe O—CO—Z dans lequel Z représente $R_3$ ou un groupe alcoxy à 1 à 4 atomes de carbone, dans un solvant inerte à des températures comprises entre —30°C et le point d'ébullition du solvant employé et éventuellement on alkyle des composés de formule I, dans laquelle $R_2$ représente un atome d'hydrogène pour donner des composés de formule I dans laquelle $R_2$ représente un groupe alkyle à 1 à 4 atomes de carbone, allyle, 2-buténylе ou 3-buténylе et/ou, dans des composés de formule I, dans laquelle $R_6$ et/ou $R_7$ représentent un atome d'hydrogène, on introduit du chlore, du brome, un groupe hydroxy ou un groupe nitro dans le cycle phénylique, et/ou, dans des composés de formule I dans laquelle $R_4$, $R_5$, $R_6$, $R_7$, $R_8$ et/ou $R_9$ représentent un groupe alcanoyloxy à 1 à 4 atomes de carbone ou alcanoylamino à 1 à 4 atomes de carbone, on hydrolyse ces groupes pour donner des groupes hydroxy ou amino et éventuellement on sépare les mélanges racémiques des composés de formule I en leurs isomères optiques et éventuellement on convertit les composés de formule I libres en leurs sels d'addition d'acides ou bien on convertit les sels d'addition d'acides en les composés libres de formule I.

10. Médicaments contenant un composé selon la revendication 1 et des excipients pharmaceutiques habituels.

**Revendications pour l'Etat contractant: AT**

1. Procédé de préparation de 2-phénylacylaminométhyl-1H-2,3-dihydro-1,4-benzodiazépines de formule I

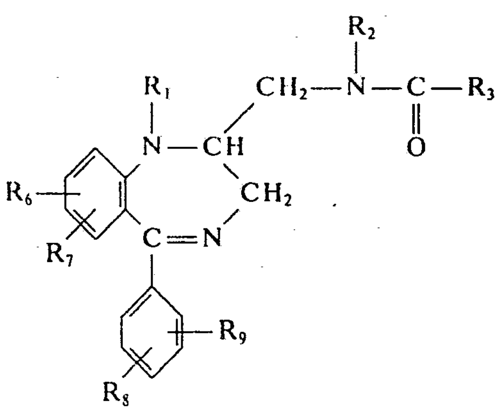

(I)

dans laquelle

$R_1$ représente un atome d'hydrogène, un groupe alkyle à 1 à 4 atomes de carbone, allyle, 2-buténylе, 3-buténylе ou cyclopropylméthyle,

$R_2$ représente un atome d'hydrogène, un groupe alkyle à 1 à 4 atomes de carbone, allyle, 2-buténylе ou 3-buténylе

$R_3$ represente un groupe de formule

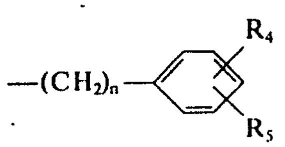

dans laquelle:

$R_4$ représente un atome d'hydrogène, un atome d'halogène, un groupe alkyle à 1 à 4 atomes de carbone, alcoxy à 1 à 4 atomes de carbone, hydroxy, alkylthio à 1 à 4 atomes de carbone, nitro, trifluorométhyle, cyano, amino, amino mono- ou disubstitué par un groupe alkyle à 1 à 4 atomes de carbone, monoalcanoylamino à 1 à 4 atomes de carbone ou alcanoyloxy à 1 à 4 atomes de carbone et

$R_5$ représente un atome d'hydrogène, un atome d'halogène, un groupe alkyle à 1 à 4 atomes de carbone, alcoxy à 1 à 4 atomes de carbone, hydroxy ou alcanoyloxy à 1 à 4 atomes de carbone ou bien

$R_4$ et $R_5$ sont liés à des atomes de carbone voisins et forment ensemble un groupe méthylènedioxy ou éthylènedioxy,

n est égal à 0,1 ou 2,

$R_6$ représente un atome d'hydrogène, un atome d'halogène, un groupe alkyle à 1 à 4 atomes de carbone, alcoxy à 1 à 4 atomes de carbone, hydroxy, alkylthio à 1 à 4 atomes de carbone, nitro, trifluorométhyle, cyano, amino, amino mono- ou disubstitué par un groupe alkyle à 1 à 4 atomes de carbone, monoalcanoylamino à 1 à 4 atomes de carbone ou alcanoyloxy à 1 à 4 atomes de carbone et

$R_7$ représente un atome d'hydrogène, un atome d'halogène, un groupe alkyle à 1 à 4 atomes de carbone, alcoxy à 1 à 4 atomes de carbone, hydroxy ou alcanoyloxy à 1 à 4 atomes de carbone, ou bien

$R_6$ et $R_7$ sont liés à des atomes de carbone voisins et forment ensemble un groupe méthylènedioxy ou éthylènedioxy,

$R_8$ représente un atome d'hydrogène, un atome d'halogène, un groupe alkyle à 1 à 4 atomes de carbone, alcoxy à 1 à 4 atomes de carbone, hydroxy, alkylthio à 1 à 4 atomes de carbone, nitro, trifluorométhyle, cyano, amino, amino mono- ou disubstitué par un groupe alkyle à 1 à 4 atomes de carbone, monoalcanoylamino à 1 à 4 atomes de carbone ou alcanoyloxy à 1 à 4 atomes de carbone, et

$R_9$ représente un atome d'hydrogène, un atome d'halogène, un groupe alkyle à 1 à 4 atomes de carbone, alcoxy à 1 à 4 atomes de carbone, hydroxy ou alcanoyloxy à 1 à 4 atomes de carbone ou bien

$R_8$ et $R_9$ sont liés à des atomes de carbone voisins et forment ensemble un groupe méthylènedioxy ou éthylènedioxy,

ainsi que leurs isomères et de leurs sels d'addition d'acides, procédé caractérisé en ce que l'on acyle des 2-aminométhyl-1H-2,3-dihydro-1,4-benzodiazépines de formule II

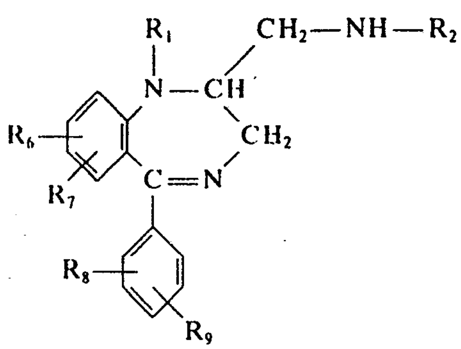

(II)

dans laquelle $R_1$, $R_2$, $R_6$, $R_7$, $R_8$ et $R_9$ ont les significations indiquées ci-dessus ou leurs sels d'addition d'acides avec un acide carboxylique ou un dérivé réactif d'acide carboxylique de formule III

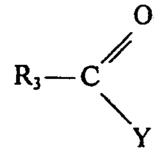

(III)

dans laquelle $R_3$ a la signification indiquée ci-dessus et Y représente un groupe hydroxy, un atome d'halogène, un groupe alcoxy à 1 à 4 atomes de carbone ou un groupe O—CO—Z dans lequel Z représente $R_3$ ou un groupe alcoxy à 1 à 4 atomes de carbone, dans un solvant inerte à des températures comprises entre —30° C et le point d'ébullition du solvant employé et éventuellement on alkyle des composés de formule I, dans laquelle $R_2$ représente l'hydrogène pour donner des composés de formule I dans laquelle $R_2$ représente un groupe alkyle à 1 à 4 atomes de carbone, allyle, 2-buténylе ou 3-buténylе et/ou, dans des composés de formule I, dans laquelle $R_6$ et/ou $R_7$ représentent l'hydrogène, on introduit un atome de chlore, ou de brome, ou un groupe hydroxy ou nitro dans le cycle phénylique, et/ou, dans des composés de formule I dans laquelle $R_4$, $R_5$, $R_6$, $R_7$, $R_8$ et/ou $R_9$ représentent des groupes alcanoyloxy à 1 à 4 atomes de carbone ou alcanoylamino à 1 à 4 atomes de carbone, on hydrolyse ces groupes pour donner des groupes hydroxy ou amino et éventuellement on sépare les mélanges racémiques des composés de formule I en leurs isomères optiques et éventuellement on convertit les composés libres de formule I en leurs sels d'addition d'acides ou bien on convertit les sels d'addition d'acides en les composés libres de formule I.

2. Procédé selon la revendication 1, caractérisé en ce que l'on prépare des 2-phénylacylaminométhyl-1H-2,3-dihydro-1,4-benzodiazépines dans lesquelles $R_1$ est un atome d'hydrogène, un groupe alkyle à 1 à 4 atomes de carbone ou un groupe cyclopropylméthyle.

3. Procédé selon la revendication 1 ou 2, caractérisé en ce que l'on prépare des 2-phénylacylaminométhyl-1H-2,3-dihydro-1,4-benzodiazépines dans lesquelles $R_2$ est un atome d'hydrogène.

4. Procédé selon la revendication 1, caractérisé en ce que l'on prépare des 2-phénylacylaminométhyl-1H-2,3-dihydro-1,4-benzodiazépines dans lesquelles:

$R_1$ représente un groupe alkyle à 1 à 4 atomes de carbone,
$R_2$ représente un atome d'hydrogène,
$R_4$ représente un atome d'hydrogène, un atome d'halogène, un groupe cyano, un groupe alkyle à 1 à 4 atomes de carbone ou alcoxy à 1 à 4 atomes de carbone,
$R_5$ représente un atome d'hydrogène, un atome d'halogène, un groupe alkyle à 1 à 4 atomes de carbone ou alcoxy à 1 à 4 atomes de carbone,
n est égal à 0,1 ou 2,
$R_6$ représente un atome d'hydrogène, un atome d'halogène, un groupe alkyle à 1 à 4 atomes de carbone ou alcoxy à 1 à 4 atomes de carbone et
$R_7$ représente un atome d'hydrogene, un atome d'halogène, un groupe alkyle à 1 à 4 atomes de carbone ou alcoxy à 1 à 4 atomes de carbone, ou bien
$R_6$ et $R_7$ sont liés à des atomes de carbone voisins et forment ensemble un groupe méthylenedioxy ou éthylènedioxy,
$R_8$ représente un atome d'hydrogène, un atome d'halogène, un groupe alkyle à 1 à 4 atomes de carbone, alcoxy à 1 à 4 atomes de carbone ou un groupe trifluorométhyle et
$R_9$ représente un atome d'hydrogène, un atome d'halogène, un groupe alkyle à 1 à 4 atomes de carbone ou alcoxy à 1 à 4 atomes de carbone.

5. Procédé selon la revendication 1, caractérisé en ce que l'on prépare la 2-méthyl-2-(4-cyanobenzoylaminométhyl)-5-(2-fluorophényl)-1H-2,3-dihydro-1,4-benzodiazépine et ses sels d'addition d'acides.

6. Procédé selon la revendication 1, caractérisé en ce que l'on prépare la 1-méthyl-2-benzoylaminométhyl-5-phényl-1H-2,3-dihydro-1,4-benzodiazépine et ses sels d'addition d'acides.

7. Procédé selon la revendication 1, caractérisé en ce que l'on prépare la 1-méthyl-8-fluoro-2-benzoylaminométhyl-5-phényl-1H-2,3-dihydro-1,4-benzodiazépine et ses sels d'addition d'acides.

8. Procédé selon la revendication 1, caractérisé en ce que l'on prépare la 1,8-diméthyl-2-(4-méthylbenzoylaminométhyl)-5-(4-fluorophényl)-1H-2,3-dihydro-1,4-benzodiazépine et ses sels d'addition d'acides.